# EUROPÄISCHE PATENTSCHRIFT

(11) **EP 3 082 890 B1**
(45) Veröffentlichungstag und Bekanntmachung des Hinweises auf die Patenterteilung: **10.11.2021**
(21) Anmeldenummer: 14811931.6
(22) Anmeldetag: 15.12.2014
(51) Int. Cl.: A61L 31/04, A61L 31/06, A61L 31/16, C08L 67/04, C08L 75/04, D01D 5/00, D01D 5/08

(54) **HERSTELLUNG VON RESORBIERBAREN POLYMERROHREN AUS FÄDEN**
PRODUCTION OF RESORBABLE POLYMER TUBES FROM THREADS
FABRICATION DE TUBES EN POLYMÈRE RÉSORBABLE À PARTIR DE FILS

(30) Priorität: 16.12.2013 EP 13197577
(43) Veröffentlichungstag der Anmeldung: 26.10.2016
(73) Patentinhaber: MeKo Laserstrahl-Materialbearbeitungen e.K., 31157 Sarstedt (DE)
(72) Erfinder: DOHSE, Jakob, 30171 Hannover (DE); HINRICHS, Bernd, 31319 Sehnde (DE)
(74) Vertreter: Arth, Hans-Lothar
(86) Internationale Anmeldenummer: PCT/EP2014/077754
(87) Internationale Veröffentlichungsnummer: WO 2015/091357

(56) Entgegenhaltungen:
- WO-A1-99/17817
- US-A1- 2004 037 813
- US-A1- 2010 070 020
- US-A1- 2011 062 638
- None

## Beschreibung

Die vorliegende Erfindung betrifft porenfreie Polymerrohre, bevorzugt aus bioresorbierbaren Polymeren, hergestellt durch gerichtete Aufwicklung mindestens eines Polymerfadens, wobei der Polymerfaden einen Durchmesser von ≤ 50 µm aufweist und der gerichtet aufgewickelte mindestens eine Polymerfaden bei oder bis maximal zu 15^{o}C oberhalb der Schmelztemperatur des mindestens einen Polymerfadens porenfrei verschmolzen ist, sowie ein Verfahren zur Herstellung dieser Polymerrohre, welche insbesondere für die Herstellung von Stents geeignet sind.

Die Implantation von Gefäßstützen wie beispielsweise Stents ist heutzutage ein gängiger interventioneller Eingriff zur Behandlung von Stenosen. Sie werden üblicherweise aus Metalllegierungen, wie nichtrostendem Edelstahl, Kobalt-Chromlegierungen oder Nickeltitan (z. B. Nitinol) hergestellt. Derartige Metallstents sind in großer Zahl bekannt und haben sich in der Praxis bewährt. Zum einen sollen derartige Metallstents aufgrund ihrer Metallstruktur und radialen Festigkeit gewährleisten, dass die Blutgefäße nach einer Aufdehnung und Stent-Implantation offen bleiben und der Blutfluss durch die Gefäße dauerhaft sichergestellt ist. Zum anderen dienen Stents in der Krebsbehandlung dazu, durch bösartige Tumoren verursachte Verengungen von Atemwegen (Luftröhre), Gallenwegen oder der Speiseröhre zu verhindern oder nach einer Aufdehnung offenzuhalten.

Stents werden derzeit in zwei Grundtypen eingeteilt: dauerhafte (biostabile) und resorbierbare (degradierbare) Stents. Dauerhafte Stents sind so ausgestaltet, dass sie im Gefäß für einen unbestimmten Zeitraum verbleiben können. Resorbierbare Stents werden hingegen über einen vorbestimmten Zeitraum hinweg im Gefäß abgebaut.

Neuere Untersuchungen haben gezeigt, dass Gefäßstenosen nicht permanent durch eine Endoprothese, insbesondere in Form eines Stents, gestützt werden müssen. Es ist vollkommen ausreichend, das Blutgefäß für einen begrenzten Zeitraum zu stützen, bis das traumatisierte Gewebe des Gefäßes verheilt und sich die glatten Gefäßmuskelzellen regeneriert haben. Sie übernehmen dann wieder die Aufgabe, das Blutgefäß offen zu halten, weshalb der Stent nicht länger erforderlich ist und nicht im Gefäßlumen verbleiben muss. Diese zeitlich begrenzte Funktion der Gefäßstützung wird durch sogenannte resorbierbare Stents erfüllt, die im Körper abgebaut werden.

Resorbierbare Stents haben den Vorteil, dass das körperfremde Material nicht dauerhaft im Gefäß verbleibt und die Gefahr einer negativen Gefäßreaktion wie einer Restenose somit zeitlich begrenzt wird. Bei Kindern besteht für resorbierbare Stents zusätzlich der Vorteil, dass die Gefäße in ihrem Wachstum nicht durch eine permanente Gefäßstütze gehemmt werden.

Derzeit sind zwei grundsätzlich unterschiedliche Materialien für die bioresorbierbaren Stents im Einsatz bzw. in der klinischen Untersuchung:
- Biopolymere, d.h. resorbierbare Polymere, insbesondere Polylactide
- Metalle, insbesondere Magnesiumlegierungen

Bezüglich der bioresorbierbaren Metall-Stents hat bislang nur die Firma Biotronik klinische Studien mit einer bioresorbierbaren Magnesium-Legierung durchgeführt. Die Firma Lifetech (China) hat hingegen erste Tierversuche mit einem eisenbasierten, resorbierbaren Stent durchgeführt. Von anderen Firmen sind keine klinischen Studien oder präklinischen Studien am Tiermodel bekannt. Obgleich grundsätzlich das angestrebte Ziel einer Resorption des implantierten Stents erreicht wird, besteht bei Magnesiumlegierungen bislang das Problem eines zeitlich nicht definierten Abbaus des Stents. Je nach Legierung ist der Materialabbau starken Schwankungen unterworfen, schwer kontrollierbar und im Allgemeinen zu schnell, um ein sicheres Einwachsen des Stents in die Gefäßwände und Übernahme der Stützfunktion bis zur Regeneration des Gefäßabschnitts zu gewährleisten. Durch eine unkontrollierte Degradation können sich Bruchstücke des Stents ablösen und Thrombosen verursachen.

Die meisten Entwicklungen resorbierbarer Stents konzentrieren sich aber auf Biopolymere und hierbei auf Polylactide, auch Polymilchsäuren genannt (kurz PLA, vom englischen Wort *polylactic acid).* Sie sind technische Biopolymere, gehören zu den Polyestern und sind aus vielen, chemisch aneinander gebundenen Milchsäuremolekülen aufgebaut.

Von den Polylactiden werden insbesondere das Poly-L-Iactid (PLLA) und das Polylactid-co-Glycolid (PLGA) für resorbierbare Stents verwendet.

Als Erste hat Kyoto Medical im Nov. 2007 eine CE-Zulassung für einen unbeschichteten PLLA Stent Igaki-Tamai^{®} erhalten. Später gelang Abbott für den Absorb^{®} die CE-Zulassung; ein PLLA Stent mit Everolimus als Wirkstoff in einer Poly-D,L-Lactid (PDLLA) Beschichtung. Im Jahr 2013 hat Elixir die Zulassung für seinen PLLA basierten Stent Desolve^{®} mit einer Novolimus enthaltenden Beschichtung erhalten.

Polylactide sind problemlos verfügbar. Es gibt mehrere Hersteller mit langjährigen Erfahrungen. Das Material gilt als körperverträglich. Aus der Chirurgie ist bioresorbierbares Polylactid-Nahtmaterial seit Jahren bekannt und im Einsatz. Folglich existieren keine Einwände Polylactide als Implantatmaterial zu verwenden und es müssen keine umfangreichen Biokompatibilitäts-Untersuchungen durchgeführt werden. Die Resorbierbarkeit lässt sich durch Wahl der geeigneten Polylactide bzw. Materialmischungen (Co-polymere) genau einstellen. Derzeit strebt man eine völlige Degradation, d.h. einen völligen Abbau der Stents bzw. Stentstreben innerhalb von 18 Monaten (1,5 Jahre) nach Implantation an.

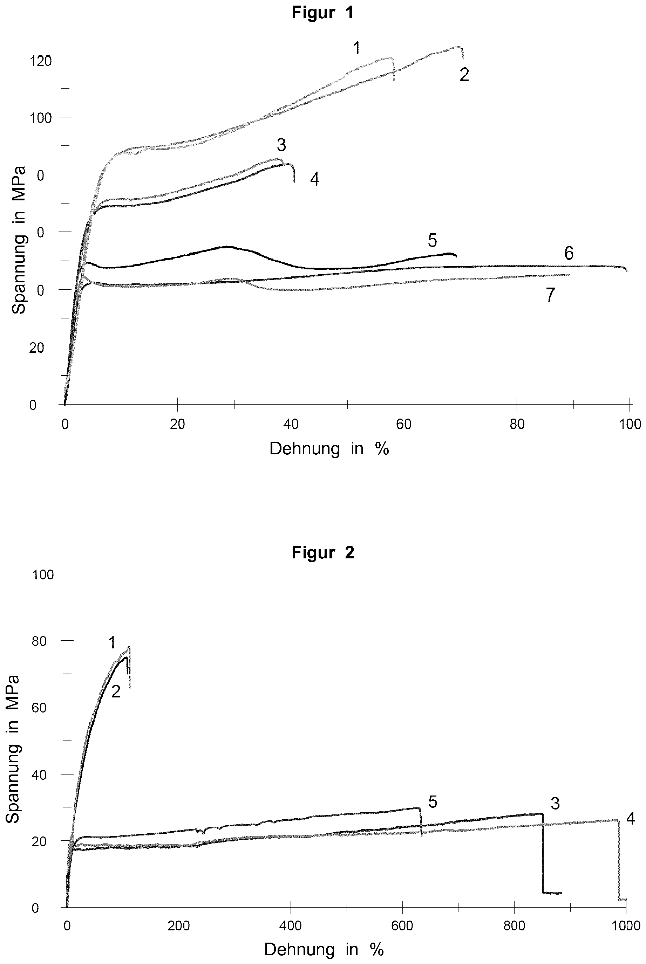

Die Polylactide PLLA und PLGA weisen als Werkstoff für Stents, das heißt in Form eines Rohrs, im Allgemeinen eine hohe Bruchdehnung auf. Bruchdehnungen bis zu 100 % für ein handelsübliches PLLA Rohr (ø 1,8 x 0,15 mm) sind problemlos erreichbar; allerdings mit dem Nachteil, dass die Festigkeit dieser Rohre nur 50 - 60 MPa beträgt (siehe Figur 1). Festigkeitssteigernde Maßnahmen führen hingegen zu einer Verringerung der Bruchdehnung. Den Vorteilen der Verwendung von Polylactiden steht somit auch eine Reihe von Nachteilen gegenüber.

Als größter Nachteil der Polylactide ist die begrenzte Festigkeit des Materials zu nennen, weshalb Verfahren zur Festigkeitssteigerung zum Einsatz kommen. PLLA Rohre zeigen bei Zugversuchen eine Festigkeit von unter 60 MPa (typischerweise 50 MPa). Aufgrund der geringen Festigkeit der Polylactide sind hohe Wandstärken bei den Stentrohlingen (Polymerrohre) respektive den Stentstreben notwendig, damit die Stents eine ausreichende radiale Festigkeit zur Gefäßstützung aufweisen.

Große Anstrengungen werden unternommen, um die Festigkeit der Polymere bzw. der Rohre zu steigern. Dennoch betragen heutzutage die Rohr- bzw. Stegwandstärken mindestens 150 µm, um eine ausreichende radiale Festigkeit zu gewährleisten. Mit diesen Wandstärken sind kleine Gefäßdurchmesser nicht erreichbar. Diese massiven Stege stehen im Widerspruch zu dem Trend, die derzeit nicht resorbierbaren Stents mit immer dünneren Stegen herzustellen mit Stegquerschnitten von teilweise nur noch 60 µm und darunter. Man erwartet von diesen filigranen Stents bessere Heilungschancen für das Gefäß.

Die festigkeitssteigernden Maßnahmen führen wiederum zu einer geringeren Bruchdehnung der Polymere, weshalb bereits geringe Überdehnungen der Polylactid-Stents zu Brüchen einzelner Stege führen. Trotz der hohen Stegquerschnitte ist die Festigkeit der Stentstreben oder auch Stege begrenzt, was in Kombination mit der geringen Bruchdehnung (der Bruchempfindlichkeit) zu einer kostenaufwändigen und langwierigen Prozedur bei Stentimplantationen führt.

So wird empfohlen, die PLLA Stents nicht direkt zu implantieren ("direct stenting"), sondern mit Hilfe einer Ballondilatation das stenotisierte Gefäß zuvor aufzudehnen ("pre-dilatation"). Teilweise wird noch eine Atherektomie durchgeführt, d.h. mittels minimal-invasiver Methoden werden die harten Ablagerungen in den Arterien entfernt. Danach soll der Gefäßdurchmesser vorzugsweise mittels der IVUS oder OCT Methode präzise vermessen werden, um die richtige Stentgröße auszuwählen und damit eine Über- oder Unterdehnung des Polylactid-Stents zu vermeiden. Schließlich wird der Polylactid-Stent dann in das bereits geöffnete und vorbereitete Gefäß eingesetzt. Eine Post-Dilatation des implantierten Stents mit einem noncompliant Ballonkatheter wird zusätzlich empfohlen.

Als weiterer Nachteil der Polylactid-Stents sei hier die begrenzte Lagerfähigkeit aufgrund der Empfindlichkeit des Materials gegenüber Temperatur und Feuchtigkeit genannt. Unerwünschte Zersetzungsprozesse können frühzeitig vor der Implantation beginnen.

Polymere allgemein neigen zudem zum Kriechen, insbesondere gedehnte Strukturen aus den Polymeren zeigen dieses Verhalten. Auf den Ballonkatheter gecrimpte Stents dehnen sich ganz langsam und allmählich wieder etwas auf, wodurch der Stent keinen festen Sitz mehr auf dem Ballonkatheter hat. Im Gefäß aufgedehnte Stents ziehen sich wieder leicht zusammen und verringern damit den Gefäßquerschnitt respektive den Blutfluss.

Rohre für die Herstellung von Gefäßimplantaten bzw. Stents weisen kleine Durchmesser von typischerweise unter 3,0 mm auf. Bestehen diese Rohre aus Biopolymeren, werden sie bevorzugt durch Extrusion hergestellt. Bei einer Herstellung durch Extrusion (Strangpressen) wird ein hochreines trockenes Polylactid-Granulat in kleinen Extrudern zu einer dickflüssigen Masse erschmolzen und durch einen Schneckentrieb kontinuierlich unter hohem Druck aus einer Düse als Rohr ausgepresst.

Ein weiteres Herstellungsverfahren ist das Spritzgießen (Spritzguss). Dazu wird mit einer Spritzgießmaschine der jeweilige Werkstoff in einer Spritzeinheit plastifiziert und in ein Spritzgießwerkzeug eingespritzt. Der Hohlraum, die Kavität, des Werkzeugs bestimmt die Form und die Oberflächenstruktur des fertigen Teils. Alternativ können Polymerrohre auch aus Hohlkörpern gezogen werden: Rohrziehprozess.

Den Herstellungsverfahren Extrusion und Spritzgießen ist gemein, dass die langkettigen Polylactid-Molekülketten weitgehend ungerichtet sind bzw. leicht in Pressrichtung (axiale Rohrrichtung) vororientiert sind und die Rohre in einem weitgehend spannungsfreien Zustand vorliegen. Die Zugfestigkeiten der Rohre sind mit 50 bis 60 MPa gering. Ausreichende radiale Festigkeiten der Stents lassen sich mit diesen Polylactid-Rohren nur durch hohe Wandstärken bzw. große Querschnitte der Stentstreben erreichen. Ein Vorteil der mittels Extrusion oder Spritzgießen hergestellten Polymerrohre sowie der daraus gewonnenen Stents ist, dass die Polymerrohre und Stents nicht porös sind, d.h. die Polymerrohre und die Stentstreben weisen keine Poren oder Zwischenräume auf, wie es bei Polymerrohren aus aufgewickelten Fäden der Fall ist und haben daher den Vorteil, sich gleichmäßig unter physiologischen Bedingungen abzubauen.

Hier weisen Polymerrohre, welche durch Aufwickeln von Polymerfäden mittels Lösungsspinnen oder Schmelzspinnen erhalten werden den Nachteil auf, dass gewebeartige oder filzartige Strukturen entstehen mit Poren oder Zwischenräumen zwischen den aufgewickelten Fäden, so dass kein gleichmäßigen biologischer Abbau unter physiologischen Bedingungen stattfindet und Bruchstücke derartiger Rohre vor allem im vaskularen Bereich Komplikationen bis hin zum Herzinfarkt verursachen können. Vorteil dieser durch Aufwicklung von Fäden erhaltenen Polymerrohre ist hingegen die Ausrichtung der Molekülketten entlang der Fadenlängsachse, wodurch höhere Zugfestigkeiten erhalten werden, so dass sich derartige Polymerrohre nur für große Körperöffnungen eignen und aus nicht bioresorbierbaren Polymeren bestehen wie beispielsweise Speiseröhrenstents in Form von einem Rohr aus aufgewickelten Fäden mit Poren, welche eine Größe haben, dass eine Krebszelle diese nicht mehr passieren kann. Ein derartiger Speiseröhrenstent ist beispielsweise in der US-Patentanmeldung US 2013103139 A1 offenbart.

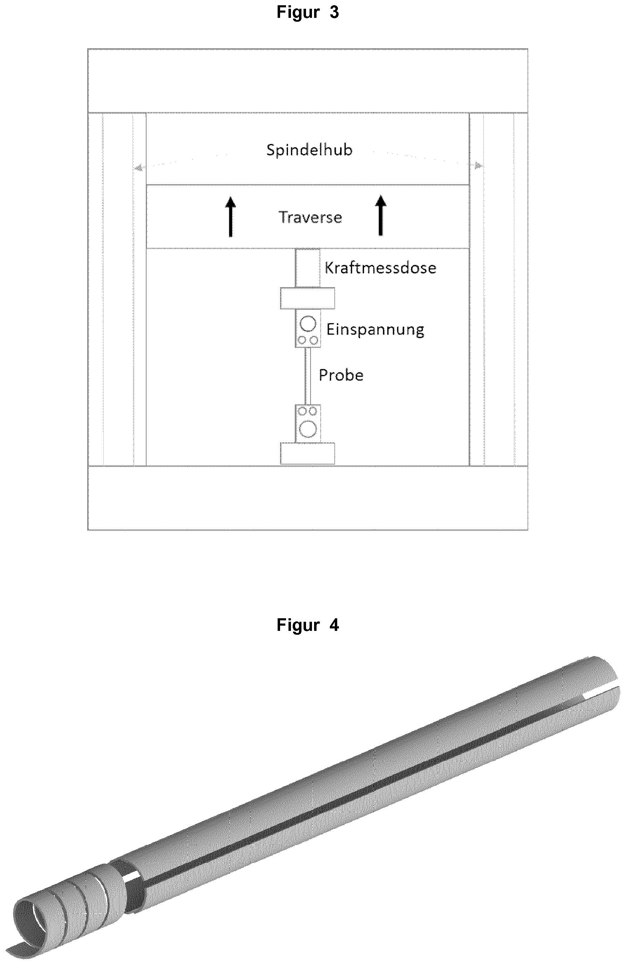

Die US-Offenlegungsschrift US 2004/0037813 A1 offenbart auf einem Substrat elektroabgeschiedenes Collagen zur Herstellung von Geweben und Organen. Figur 3 von US 2004/0037813 A1 zeigt eine Vorrichtung zur Aufbringung des Collagens auf ein rotierendes Substrat und Figur 10 zeigt eine Vergrößerung einer elektrogesponnenen Matrix aus Collagen Typ I und Typ III im 50:50-Gemisch. Figur 12 zeigt eine Vergrößerung eines Filzes aus Collagen Typ II und die Figuren 14 und 15 zeigen einen Stent vor und nach der Beschichtung mit Collagennanofasern. Die Figuren 10, 12 und 15 von US 2004/0037813 A1 zeigen eine filzartige Struktur aus ungeordneten Collagenfasern. Weder eine gerichtete Aufwicklung der Collagenfasern noch ein porenfreies Verschmelzen der aufgebrachten Collagenfasern wird in US 2004/0037813 A1 offenbart. Gemäß der in US 2004/0037813 A1 offenbarten Verfahren kann keine gerichtete Aufwicklung eines Polymerfadens erfolgen und auch kein porenfreies Verschmelzen der Collagenfasern.

Die internationale Offenlegungsschrift WO 99/17817 A1 offenbart ein poröses Gewebe aus ungeordneten Filamenten sowie eine Vorrichtung und ein Verfahren zur Herstellung dieser Gewebe. Figur 1 von WO 99/17817 A1 zeigt die Vorrichtung und Figur 2 das Gewebe aus einem Gewirr ungeordneter Fäden. Weder ein porenfreies Polymerrohr noch eine gerichtete oder geordnete Ausrichtung der Polymerfäden werden in WO 99/17817 A1 offenbart. WO 99/17817 A1 beschreibt auch kein porenfreies Verschmelzen der Polymerfäden sondern ein nicht-porenfreies Verschmelzen der Polymerfäden unterhalb der Schmelztemperatur.

Die US-Offenlegungsschrift US 2010/0070020 A1 offenbart einen aus Polyurethanfasern elektrogesponnenen Grundkörper, der dann mit einem Stützgewebe ummantelt und nochmals mit einer äußeren Schicht umrundet wird. Figur 2A von US 2010/0070020 A1 zeigt eine elektronenmikroskopische Aufnahme elektrogesponnener willkürlich ausgerichteter Polymerfasern. Weder ein porenfreies Polymerrohr noch eine gerichtete oder geordnete Ausrichtung der Polymerfäden werden in US 2010/0070020 A1 offenbart.

Der Stand der Technik offenbart kein Polymerrohr aus gerichtet aufgewickelten Polymerfäden, welche danach porenfrei verschmolzen wurden, so dass sich ein massives porenfreies Polymerrohr wie bei einem Extrusions- oder Spritzgießverfahren ergibt, jedoch mit dem erfindungsgemäßen Unterschied der Beibehaltung der Ausrichtung der Polymerketten oder Molekülketten in den Polymerfäden, wodurch gesteigerte Zugfestigkeiten erhalten werden und eine Rückstellkraft in Richtung Nominaldurchmesser des Polymerrohres erhalten wird.

Bei den mittels Extrusion oder Spritzgießen hergestellten Polymerrohren müssen hingegen festigkeitssteigernde Maßnahmen vorgenommen werden, weil die Ausrichtung der Molekülketten fehlt. Zudem ist bekannt, dass aufgrund der Scherkräfte bei einem Extrusionsverfahren das durchschnittliche Molekulargewicht des eingesetzten Polymers abnimmt, weil Molekülketten gespalten werden. Beim Lösungsspinnen bleibt das **Molekulargewicht** der Polymere erhalten. In den konkurrierenden Verfahren der Extrusion und im Spritzguss nimmt das Molekulargewicht um 30 - 50% ab. Das liegt primär an den hohen Scherkräften in der Extruderschnecke und sekundär an der hohen thermischen Belastung, die bei der Plastifizierung auf das Polymer ausgeübt wird. Gemessen werden kann das Molekulargewicht mithilfe der Viskosimetrie durch die Bestimmung der Inhärenten Viskosität (IV). Dabei wird die Durchlaufzeit eines gelösten Polymers mit der Durchlaufzeit des reinen Lösungsmittels verglichen. Ein typisches Ausgangsmaterial wie PL38 von Corbion Purac hat eine IV von 3,8 dl/g. Im Extruder nimmt die IV dann auf 2,5 dl/g ab. Im Nachgang beim Laserschneiden (ca. 5% IV Abnahme) und der Sterilisation (ca. 25% IV Abnahme) verliert das Polymer weiter an Molekulargewicht. Bei den erfindungsgemäßen Verfahren nimmt das Molekulargewicht während der Herstellung des Polymerrohres kaum ab (unter 5%). Das höhere Molekulargewicht wirkt sich positiv auf die mechanischen Eigenschaften aus und verlängert auch die Resorptionszeit, weil längere Ketten häufiger gespalten werden müssen.

Von thermoplastischen Kunststoffen ist bekannt, dass die Materialfestigkeiten durch Verstrecken gesteigert werden können. Dieses Verfahren wird standardmäßig vielfach in der industriellen Fertigung von Kunststoffprodukten angewandt.

Beim Verstrecken gleiten die langkettigen Polymermoleküle aufeinander, werden entknäuelt und in Dehnrichtung zueinander ausgerichtet. Sie liegen zunehmend parallel zueinander. Die Orientierung der Molekülketten ist gleichbedeutend mit einer partiellen Kristallisation (Teilkristallisation), wobei im Material gleichzeitig Spannungen induziert werden (innere Spannungen). Die teilkristalline Anordnung sollte durch Sekundärbindungen (Van der Waals Kräfte, Dipol-Dipol-Bindungen oder Wasserstoffbrücken) stabilisiert werden, um den Spannungsabbau, d.h. die Tendenz zum Kriechen in Richtung der Ursprungsform zu vermeiden. Das Verstrecken muss nicht zwingend bei Raumtemperatur erfolgen. So kann das Verstrecken von beispielsweise PLLA-Rohren bei Temperaturen von typischerweise 50°C bis 100°C erfolgen.

Dies bedeutet, dass beim Verstrecken zwei Prozesse gleichzeitig ablaufen: die Moleküle werden in Dehnrichtung ausgerichtet und die entstehenden kristallinen Bereiche werden gleichgerichtet.

Die Polymerrohre können zum Verstrecken über einem Dorn geweitet werden, so dass eine Festigkeitssteigerung eintritt. Eine andere Methode kommt aus der Ballonkatheterherstellung. Mit den gleichen Anlagen und Vorrichtungen zum Aufblasen (Ausformen) eines Ballons für einen Ballonkatheter wird das PLLA-Rohr in einer Form auf einen größeren Durchmesser radial aufgedehnt. Ein ähnliches Verfahren wird in US2011/0062638 A1 offenbart. Figur 2 zeigt das Spannungs-Dehnungsdiagramm eines mit diesem Verfahren radial aufgedehnten, d.h. verfestigten PLLA Rohres aus dem Stand der Technik. Es ist eine deutlich höhere Festigkeit, aber auch die verringerte Bruchdehnung zu erkennen. Die geringere Bruchdehnung führt zu einer begrenzten Dehnbarkeit der Polylactid-Stents und zu einer Bruchgefahr bei nur geringer Überdehnung über den Nominaldurchmesser des Stents. Der **Nominaldurchmesser** eines Stents bezeichnet den Innendurchmesser des Stents im aufgedehnten (dilatierten), implantierten Zustand.

Wie dargelegt weisen Polymere wie beispielsweise Polylactide als Stentmaterial den entscheidenden Nachteil einer begrenzten Festigkeit auf, weshalb Verfahren zur Festigkeitssteigerung zum Einsatz kommen. Allerdings führen die derzeitigen, festigkeitssteigernden Methoden wie z.B. das Verstrecken zu einer begrenzten Dehnbarkeit und damit zu einer starken Einschränkung bei der Verwendung der Biopolymerstents.

Ziel der vorliegenden Erfindung ist es daher, durch geeignete Maßnahmen und Verfahren die Festigkeit von Polymerrohren und daraus hergestellten Stents oder anderen Gefäßimplantaten zu steigern und Polymerrohre sowie Stents mit verbesserten Zugfestigkeiten und Rückstellkräften zum Nominaldurchmesser bereitzustellen. Die erfindungsgemäße Herstellung von Polymerrohren beseitigt die starken Einschränkungen bzw. verbessert die Eigenschaften der Polymerrohre, die dadurch für die Herstellung von Stents besonders geeignet sind.

Aufgabe der vorliegenden Erfindung ist es ein Polymerrohr bereitzustellen, welches eine erhöhte radiale Festigkeit bei kaum verminderter Bruchdehnung aufweist und daher für die Herstellung von Stents besonders geeignet ist.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die vorliegende Erfindung ist gerichtet auf ein porenfreies **Polymerrohr** und vorzugsweise auf ein bioresorbierbares porenfreies Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden bei oder bis maximal zu 15^{o}C oberhalb der Schmelztemperatur des mindestens einen Polymerfadens porenfrei verschmolzen ist. Der Durchmesser des erfindungsgemäß genutzten Polymerfadens ist ≤ 50 µm und bevorzugt ≤ 30 µm, weiter bevorzugt ≤ 20 µm, weiter bevorzugt ≤ 15 µm, weiter bevorzugt ≤ 12 µm, weiter bevorzugt ≤ 10 µm, weiter bevorzugt ≤ 8 µm, noch weiter bevorzugt ≤ 5 µm und besonders bevorzugt ≤ 2,5 µm. Der Durchmesser des Polymerfadens liegt bevorzugt in folgenden Bereichen: zwischen 50 nm und 50 µm, weiter bevorzugt zwischen 100 nm und 20 µm, weiter bevorzugt zwischen 150 nm und 10 µm, noch weiter bevorzugt zwischen 200 nm und 5 µm und besonders bevorzugt zwischen 300 nm und 2,5 µm. Ein weiterer Aspekt ist ein Polymerrohr hergestellt durch Aufwickeln von mindestens einem Polymerfaden, wobei der Polymerfaden einen Durchmesser im Bereich von 50 nm bis 50 µm aufweist.

Unter dem Begriff "porenfrei" wie hierin verwendet, soll verstanden werden, dass das erfindungsgemäße Polymerrohr keine Poren aufweise wie sie beispielsweise in Geweben oder Filzen oder Gespinsten von ungeordneten oder willkürlich ausgerichteten Fäden vorkommen. Die Fäden liegen ungeordnet aufeinander und die Zwischenräume zwischen den Polymerfäden werden als Poren bezeichnet. Selbst bei einem Verschmelzen dieser filzartigen oder gewebeartigen oder gespinstartigen Strukturen können die Poren nicht vollständig beseitigt werden, außer die Polymerfäden in diesen Strukturen werden vollständig verflüssigt, was jedoch auch die filzartigen oder gewebeartigen oder gespinstartigen Strukturen vollständig zerstört und daher widersinnig wäre. Somit wird unter dem Begriff "porenfrei" eine Struktur des erfindungsgemäßen Polymerrohrs verstanden, welche keine Poren aufweist, wobei als **Pore** ein Zwischenraum mit einem Volumen von mindestens **900 nm³** oder einem Maximaldurchmesser von mindestens 800 nm bezeichnet wird. Als Maximaldurchmesser wird der größtmögliche Durchmesser in einer Pore beliebiger geometrischer Ausgestaltung bezeichnet. Poren beziehen sich dabei auf das innere Volumen des Rohres und nicht auf evtl. Unebenheiten an der Materialoberfläche, d.h. der inneren als auch äußeren Oberfläche des Polymerrohres.

Der Begriff "porenfrei" wird darüber hinaus wie folgt definiert: Ein herkömmlich extrudiertes Rohr gleichen Materials wird im Außendurchmesser und in der Wandstärke vermessen. Über den gemittelten Außendurchmesser und die gemittelte Wandstärke, sowie die gemessene Länge des Rohrs wird das Volumen ermittelt. Weiterhin wird die Masse des Rohrs auf einer Feinwaage bestimmt. Die Masse des Rohrs wird durch das Volumen geteilt. Dadurch ergibt sich die mittlere Dichte des Rohrs. Das erfindungsgemäß hergestellt Rohr wird in gleicher Weise vermessen und ebenfalls die mittlere Dichte bestimmt. Sofern die mittlere Dichte des extrudierten Polymerrohrs maximal 5%, bevorzugt nicht größer als 3% größer als die mittlere Dichte des erfindungsgemäß hergestellten Rohrs ist, so wird das erfindungsgemäße Rohr als "porenfrei" bezeichnet. Ein Polymerrohr ist daher "porenfrei", wenn seine mittlere Dichte nicht größer als 5%, bevorzugt nicht größer als 3% der mittleren Dichte eines mittels Extrusion hergestellten Polymerrohres gleichen Materials ist.

Somit betrifft die vorliegende Erfindung ein porenfreies Polymerrohr und vorzugsweise ein bioresorbierbares porenfreies Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden bei oder bis maximal zu 15^{o}C oberhalb der Schmelztemperatur des mindestens einen Polymerfadens porenfrei verschmolzen ist. Das erfindungsgemäße porenfreie und vorzugsweise bioresorbierbare Polymerrohr besteht somit nicht aus einem Fadengewirr und besteht nicht aus einem Gewebe und besteht nicht aus einem Filz. Zudem bilden die gerichtet aufgewickelten Polymerfäden das Polymerrohr. Die Polymerfäden werden nicht um ein Substrat oder ein Medizinprodukt wie z.B. einen Stent und vor allem einen Metallstent gewickelt, auf dem die aufgewickelten Polymerfäden oder das erhaltene Polymerrohr verbleiben sollen. Die erfindungsgemäße Verwendung eines Dorns oder einer Spule zum Aufwickeln der Polymerfäden dient nur dazu, dem Polymerrohr einen definierten Innendurchmesser, nämlich bereits den Nominaldurchmesser zu verleihen und ein gerichtetes Aufwickeln zu ermöglichen, wobei das fertige Polymerrohr natürlich von dem Dorn oder der Spule abgenommen wird. Die erfindungsgemäßen Polymerrohre umfassen daher kein Stützgerüst wie beispielsweise einen Stent, um den herum sich das Polymerrohr befindet. Das erfindungsgemäße Polymerrohr als auch die daraus vorzugsweise mittels Laser geschnittene Struktur bilden selbst den Stent.

Ferner betrifft die vorliegende Erfindung ein porenfreies und vorzugsweise bioresorbierbares Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden bei oder bis maximal zu 15^{o}C oberhalb der Schmelztemperatur des mindestens einen Polymerfadens porenfrei verschmolzen ist. Der Begriff "gerichtet" wie hierin verwendet bezeichnet eine geordnete Aufwicklung des Polymerfadens oder der Polymerfäden. Eine gerichtete oder geordnete Aufwicklung des Polymerfadens oder der Polymerfäden kann die Aufwicklung nach einem bestimmten Muster bedeuten oder das Vorliegen einer bestimmten Ordnung zumindest in Teilbereichen oder Schichten des Polymerrohrs. Bevorzugt ist eine Aufwicklung wie ein Kabel auf einer Kabeltrommel. Eine gerichtete oder geordnete Aufwicklung schließt nicht aus, dass sich Polymerfäden kreuzen oder übereinander liegen, wobei in gewissen Abschnitten oder Lagen oder Schichten des Polymerrohrs die aufgewickelten Polymerfäden parallel zueinander liegen sollten. Nicht gerichtet oder nicht geordnet ist ein Aufbringen einer Lösung von Polymerfäden mittels Sprühvorrichtung oder Elektrospinning wie z.B. in US 2010/0070020 A1, WO 99/17817 A1, US 2004/0037813 A1 oder US 2013103139 A1 offenbart, weil sich dabei willkürliche Ausrichtungen der Polymerfäden ergeben. Da jedoch die Molekülketten oder synonym als Polymerketten bezeichnet, in dem Polymerfaden bereits entlang der Längsachse des Polymerfadens orientiert sind, kann diese Orientierung ausgenutzt werden, um vorteilhafte Eigenschaften zu erzeugen, wenn die Polymerfäden ebenfalls in einer Ordnung oder Orientierung aufgewickelt oder zu einem Polymerrohr zusammengefügt werden. Das Gegenteil einer gerichteten oder geordneten Ausrichtung ist somit die willkürliche oder ungeordnete Ausrichtung.

Dem Begriff "Aufwickeln" oder "Aufwicklung" wie hierin verwendet, kommt eine besondere Bedeutung zu. Um den mindestens einen Polymerfaden gerichtet aufwickeln zu können, wird der Polymerfaden von der Spindüse oder der Elektrospindüse abgezogen. Die Spindüse sprüht daher nicht den Polymerfaden oder die Polymerfäden auf den Dorn oder die Spule, wodurch ein Aufwickeln und erst recht ein gerichtetes Aufwickeln unmöglich ist, sondern produziert kontinuierlich einen Polymerfaden, den man als einen Endlos-Polymerfaden bezeichnen kann, der aufgewickelt wird. Der Polymerfaden oder Endlos-Polymerfaden verbindet sozusagen die Spindüse mit dem Dorn oder der Spule. Auf dem Dorn oder der Spule wird daher ein Endlos-Polymerfaden aufgewickelt. Es werden hingegen keine Vielzahl an Fadenstücken, verknäuelte Fäden, Fadengewirre oder ein Filz auf den Dorn aufgebracht oder gesprüht oder abgeladen, was nicht gerichtet erfolgen kann.

Zudem betrifft die vorliegende Erfindung ein bioresorbierbares porenfreies Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden unter Beibehaltung der Ausrichtung der Molekülketten entlang der Fadenlängsachse bei oder bis maximal zu 15°C oberhalb der Schmelztemperatur des mindestens einen Polymerfadens verschmolzen ist oder genauer gesagt porenfrei verschmolzen ist.

Alternativ betrifft die vorliegende Erfindung ein porenfreies Polymerrohr und bevorzugt ein bioresorbierbares porenfreies Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden unter Beibehaltung der Ausrichtung der Molekülketten entlang der Fadenlängsachse bei oder bis maximal zu 15°C oberhalb der Schmelztemperatur des mindestens einen Polymerfadens porenfrei verschmolzen ist.

Erfindungsgemäß werden die gerichtet aufgewickelten Polymerfäden verschmolzen, so dass die Poren entfernt werden und ein porenfreies Polymerrohr resultiert, wobei die Verschmelzung bei oder bis maximal zu 15°C, weiter bevorzugt bis maximal zu 10°C oberhalb der Schmelztemperatur der Polymerfäden und zudem vorzugsweise derart schnell erfolgt, dass die Ausrichtung der Molekülketten entlang der Fadenlängsachse erhalten bleibt. Somit erhält man ein porenfreies massives Polymerrohr wie nach einem Extrusionsverfahren oder Spritzgußverfahren aber mit dem großen Unterschied, dass die Ausrichtung der Molekülketten entlang der Längsachse des Polymerfadens erhalten bleibt. Eine derartige Ausrichtung oder Orientierung der Molekülketten oder der Polymerketten kann jedoch durch ein Extrusionsverfahren oder Spritzgußverfahren nicht erhalten werden.

Die erfindungsgemäßen Polymerrohre weisen bevorzugt einen Innendurchmesser von 0,25 - 20 mm, weiter bevorzugt 0,5 - 15 mm, noch weiter bevorzugt von 1 ― 10 mm und besonders bevorzugt von 1,5 ― 5 mm auf. Beispielsweise werden für Stents der koronaren Gefäße typischerweise Rohre mit einem Innendurchmesser von 2,0 bis 4,0 mm verwandt mit Wandstärke von 100 bis 200 µm. Entsprechend betragen die Außendurchmesser der Polymerrohre 2,2 bis 4,4 mm.

Das Rohr und der entsprechende Faden sind aus polymerem Material und vorzugsweise aus einem resorbierbaren Polymer hergestellt. Die Polymerrohre besitzen bevorzugt einen kreisrunden Querschnitt. Der Begriff "Wand" oder "Rohrwand", wie hierin verwendet, bezeichnet die Mantel- bzw. Zylinderfläche des Polymerrohrs und somit den durch Aufwickeln mindestens eines Polymerfadens erhaltenen Anteil des Polymerrohrs ohne eventuell zusätzlich aufgebrachte Beschichtungen. Ein erfindungsgemäßes Polymerrohr weist nur eine nahtlose Wand auf. Der Begriff "Wanddicke" oder auch "Wandstärke", wie hierin verwendet, bezeichnet die Differenz der inneren und der äußeren Abmessungen, also des Außen- und des Innendurchmessers des Rohres.

Der Begriff "resorbierbar" oder "bioresorbierbar" wie hierin verwendet bedeutet, dass das Polymer, respektive das Polymerrohr, oder der daraus hergestellte Stent, sich über eine gewisse Zeit in einem menschlichen oder tierischen Organismus langsam auflöst und irgendwann nur noch dessen Abbauprodukte im Körper vorliegen und vorzugsweise vom Körper ausgeschieden werden. Zu diesem Zeitpunkt sind feste Bestandteile oder Fragmente des Polymers nicht mehr vorhanden. Die Abbauprodukte sollten physiologisch weitgehend unbedenklich sein.

Erfindungsgemäß ist es bevorzugt, wenn das Polymerrohr aus mindestens einem thermoplastischen Polymer und weiter bevorzugt aus einem thermoplastischen bioresorbierbaren Polymer hergestellt ist. Thermoplastische bioresorbierbare Polymere können aus der folgenden Gruppe ausgewählt werden bestehend aus oder umfassend:
Poly(ε-caprolacton), Polyurethan, Polyhydroxybutyrate, Polylactonsäure, Polyglycolsäure, Polylactide, Polyglycolide, Copolymere der Polylactide und
Polyglycolide, Polydioxanone, Polyethylenglycol (PEG), Polypropylencarbonat (PPC), Poly(desaminotyrosyl-tyrosine-ethylestercarbonat) (PDTE Carbonat).

Beispiele für nicht bioresorbierbare Polymere sind Nylon, Polymethylmethacrylat (PMMA), Polyamide (PA), Polycarbonat (PC), Polyethylenterephthalat (PET), Polypropylen (PP), Polystyren (PS), Polyethylen (PE), Silicone, Polytetrafluorethylen (PTFE), Polyvinylchlorid (PVC).

Da die Anwendung des Lösungsspinnens oder Elektrospinnens bevorzugt ist, sollten die bioresorbierbaren Polymere in einem organischen Lösungsmittel wie beispielsweise Aceton, Methanol, Ethanol, THF, Methylenchlorid, Benzol, Toluol, Chloroform, Tetrafluorethylen (TFE), Tetrachlorkohlenstoff, Ethylacetat, Diethylether, Acetonitril, 3-Pentanon, Butanon, Propanol, Hexafluoroisopropanol (HFIP) löslich sein. Ausgeschlossen von der vorliegenden Erfindung, weil ungeeignet, ist Collagen. Bevorzugt ist hingegen Polylactid wie z.B. PLLA sowie Copolymere aus Polylactid und Polyglycolid wie z.B. PLGA.

Ein weiterer Aspekt der Erfindung bezieht sich auf porenfreie Polymerrohre hergestellt durch gerichtete Aufwicklung mindestens eines Polymerfadens, wobei der Polymerfaden einen Durchmesser vorzugsweise im Bereich von 50 nm bis 50 µm aufweist und die Polymerfäden aus unterschiedlichen, vorzugsweise bioresorbierbaren Polymeren bestehen. Ein weiterer Aspekt der Erfindung bezieht sich auf Polymerrohre hergestellt durch gerichtete Aufwicklung mehrerer Polymerfäden, wobei die Polymerfäden einen Durchmesser vorzugsweise im Bereich von 50 nm bis 50 µm aufweisen und die Polymerfäden aus unterschiedlichen, vorzugsweise bioresorbierbaren Polymeren bestehen. Die erfindungsgemäße Herstellung der Polymerrohre durch gerichtete Aufwicklung von Polymerfäden lässt die Kombination von unterschiedlichen Polymeren oder Copolymeren in den Polymerrohren zu (im weiteren Hybrid-Rohre genannt), wobei die unterschiedlichen Polymere oder Copolymere in einer gezielten und für das Endprodukt vorteilhaften Anordnung vorliegen können. Dadurch können dem erfindungsgemäßen Polymerrohr gezielt Eigenschaften verschiedener Polymere oder Copolymere verliehen werden. Mit der Wahl der unterschiedlichen Polymere sind daher Hybridrohre herstellbar, die genau auf die Verwendung abgestimmt werden. Erfindungsgemäß bevorzugt ist die Verwendung zur Herstellung von Stents oder anderer Gefäßprothesen. In diesem Fall können die Polymerrohre genau auf die physiologischen Eigenschaften der humanen Gefäße bzw. die Heilungsprozesse und Degradationsvorgänge abgestimmt werden.

So können die Rohre z.B. lagenweise aus unterschiedlichen Polymeren bestehen. Die innere (dem Lumen zugewandte) und äußere (der Gefäßwand zugewandte) Schicht der Wand eines erfindungsgemäßen Polymerrohres kann bevorzugt aus einem Polymer mit sehr langsamer Degradation bestehen, während dazwischen im Inneren der Wand (im Inneren des Rohrquerschnittes) ein Polymer mit hoher Festigkeit (aber gegebenenfalls schneller Degradation) verwendet wird.

Neben der lagenweisen Kombination unterschiedlicher Polymere in der Wand der Polymerrohre (radiale Polymerkombinationen) sind erfindungsgemäß auch Polymerrohre herstellbar, die über die Rohrlänge unterschiedliche Polymere und damit unterschiedliche Eigenschaften aufweisen (axiale Polymerkombinationen). Das heißt die unterschiedlichen Polymere können sowohl in Schichten als auch in Ringen in der Wand des Polymerrohrs variieren bzw. angeordnet sein.

Jegliche Kombinationen von unterschiedlichen Polymeren in radialer und axialer Richtung sind möglich. Dabei entspricht es einer Ausführungsform, dass die Polymerrohre aus einem Polymerfaden hergestellt werden und der Polymerfaden aus unterschiedlichen Polymeren besteht. Die Erfindung umfasst aber auch Polymerrohre, hergestellt aus mindestens zwei Polymerfäden bestehend aus unterschiedlichen Polymeren, bevorzugt aus resorbierbaren Polymeren.

Es ist des Weiteren bevorzugt, wenn die erfindungsgemäßen Polymerrohre eine Wandstärke ≤ 250 µm (z.B. für periphere Gefäße), weiter bevorzugt ≤ 200 µm, weiter bevorzugt ≤ 150 µm (z.B. für koronare Gefäße), und besonders bevorzugt ≤ 100 µm aufweisen. Ein weiterer Aspekt der Erfindung betrifft Polymerrohre, die über die Rohrlänge zu unterschiedlichen Wandstärken aufgewickelt werden. Diese Wandstärken sollen den Festigkeitsbeanspruchungen der, aus den erfindungsgemäßen Polymerrohren hergestellten, Bauteilen entsprechen.

Die erfindungsgemäßen Polymerrohre weisen mindestens 5, bevorzugt mindestens 10 und noch weiter bevorzugt mindestens 20 Schichten bzw. Lagen von Polymerfäden auf. Dabei besteht eine Schicht aus den Wicklungen des mindestens einen Polymerfadens, die in einer Ebene verlaufen, die parallel zur Achse des Polymerrohrs liegt, d. h. die Dicke einer Schicht entspricht dem Durchmesser des verwendeten Polymerfadens.

Bei den erfindungsgemäß hergestellten porenfreien Polymerrohren weisen die Polymere vorzugsweise besonders lange Molekülketten auf, welche zudem entlang der Fadenlängsachse ausgerichtet sind, insbesondere wenn die Fäden durch Lösungsspinnen hergestellt werden. Dies ist für die Festigkeit und eine langsamere Degradation der Polymerrohre von Vorteil. Durch die Ausrichtung der Molekülketten entlang der Faserlängsachse baut sich bei Deformation des Polymerrohres oder eines aus dem Polymerrohr hergestellten Stents eine Rückstellkraft auf, welche darauf gerichtet ist, das Rohr bzw. den Stent wieder auf den Außendurchmesser des Dorns für die Aufwicklung zu bringen, d.h. auf den Nominaldurchmesser. Dabei entspricht der Außendurchmesser des Dorns dem Innendurchmesser des Polymerrohrs oder des Stents nach dem Verschmelzen der ausgerichteten Molekülketten. Es ist erfindungsgemäß, dass das Polymerrohr und damit auch der aus dem Polymerrohr vorzugsweise durch Laserschneiden hergestellte Stent im Nominaldurchmesser hergestellt werden, d.h. der Außendurchmesser des Dorns dem Innendurchmesser des Polymerrohrs nach der Dilatation entspricht, damit beim gekcrimpten Polymerrohr oder beim geckrimpten Stent eine Rückstellkraft in Richtung des Nominaldurchmessers aufgebaut wird. Im Stand der Technik werden derartige Polymerrohre durch Extrudieren hergestellt, wobei bei der Extrusion keine Ausrichtung der Molekülketten erfolgt, wodurch extrudierte Rohre im Vergleich zu den erfindungsgemäßen Rohren deutlich geringere Zugfestigkeiten und Bruchdehnungen aufweisen und sich aufgrund der ungeordneten und willkürlichen Anordnung der Molekülketten keine oder nur eine deutlich geringere Rückstellkraft in Richtung Nominaldurchmesser aufbaut.

Eine bevorzugte Ausführungsform der Erfindung betrifft ein Polymerrohr, welches mindestens einen **Wirkstoff** umfasst. Bevorzugt ist dabei, dass sich der mindestens eine Wirkstoff in der Wand des Polymerrohrs befindet. Es handelt sich dabei bevorzugt um Wirkstoffe, die zur Verminderung einer Restenose geeignet sind. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-α-Hydroxy-Paclitaxel, Baccatin oder andere Taxotere, Biolimus A9, Myolimus, Novolimus, Pimecrolimus, Tacroliums, Temsirolimus, Zotarolimus, Everolimus, Ridaforolimus oder weiteren sogenannten "Limus"-Derivate (auch Rapalogs genannt).

Es können auch Wirkstoffkombinationen verwendet werden. Dabei kann die Konzentration der Wirkstoffe an verschiedenen Stellen der erfindungsgemäß hergestellten Polymerrohre variieren, so kann an den Enden des Rohrs mehr Wirkstoff enthalten sein als im mittleren Bereich. Die Wirkstoffkonzentration kann auch im Verlauf der Wandstärke variieren und zum Beispiel einen Gradienten aufweisen, so dass auf der äußeren Oberfläche (abluminale Oberfläche) des Polymerrohrs mehr Wirkstoff vorliegt als auf der Oberfläche im Inneren des Rohrs (luminale Oberfläche) oder umgekehrt.

Zur besseren **Röntgensichtbarkeit** der implantierten Stents im Körper werden entweder Werkstoffe mit höherer Dichte, d.h. höherer Röntgensichtbarkeit für die Herstellung der Stents verwandt (z.B. beim Boston Scientific "Element"-Stent der Werkstoff PtCr) oder bevorzugt sogenannte Röntgenmarker in die proximalen und distalen Enden der Stents eingesetzt. Die Röntgenmarker bestehen z.B. aus Tantal, Platin oder seltener Gold, werden in kleine Markerösen eingecrimpt und häufig auch zusätzlich laserverschweißt.

Bei den erfindungsgemäßen Polymerrohren bestehen mehrere Möglichkeiten, die Röntgensichtbarkeit herzustellen. Die **Röntgenmarker** können als kleine runde Pellets oder Scheiben (ähnlich den derzeitigen Röntgenmarkern) oder auch als dünne Folien oder Drähte beim Wickeln der Rohre mit in die Rohrwand eingearbeitet, d.h. eingebunden werden.

Die bevorzugte Methode ist aber, bereits bei der Herstellung des dünnen Polymerfadens **Röntgenmarker** wie z.B. Pulver aus röntgensichtbaren Werkstoffen wie Wolfram, Tantal, Platin oder Gold in den Faden einzumischen. Beim Aufwickeln werden die röntgensichtbaren Werkstoffe somit über die gesamte Rohrwandstärke oder bevorzugt nur in bestimmte Schichten und Lagen und/oder bestimmten Bereichen des Polymerrohres eingebaut. Die Pulver haben eine Partikelgröße von kleiner 10 µm, vorzugsweise kleiner 5 µm und noch weiter bevorzugt kleiner 1 µm. Die Pulver bzw. die Röntgenmarker werden bei der Degradation des implantierten Polymerstents herausgelöst. Aufgrund ihrer Partikelgröße und ihrer Bioverträglichkeit (mikrobiellen Inertheit) sind die Röntgenmarker für den menschlichen Organismus unbedenklich.

Bei Einbringung der röntgensichtbaren Pulver oder der Röntgenmarker z.B. nur in den inneren Kern der Polymerrohrwand, wird sichergestellt, dass bis zur nahezu vollständigen Degradation des Stents die Röntgensichtbarkeit erhalten bleibt. Werden die röntgensichtbaren Pulver dagegen nur in die äußeren Randschichten der Rohre (nicht in den Kern der Rohrwand) eingebracht, kann der Degradationsfortschritt, d.h. der Abbau der Stents z.B. anhand der verbliebenen Röntgensichtbarkeit ermittelt werden.

Eine weitere Ausführungsform ist, dass die röntgensichtbaren Pulver oder die Röntgenmarker nicht über die gesamte Rohrlänge, sondern nur in gewissen Abständen ringförmig eingebracht werden. Die Abstände der röntgensichtbaren Ringe sollten vornehmlich der Stentlänge entsprechen, die aus den Rohren lasergeschnitten wird. Auf diese Weise erhält man Stents mit einer Röntgensichtbarkeit nur an den proximalen und distalen Stentenden.

Jede andere mögliche Ausführungsform, d.h. Einbringung der röntgensichtbaren Pulver oder der Röntgenmarker in das Polymerrohr ist denkbar und möglich.

Anstelle oder zusätzlich zu den aufgeführten Wirkstoffen oder Röntgenmarkern können auch **Partikel oder Nanopartikel** mit physikalischen (z.B. magnetischen oder radioaktiven) und/oder chemischen (z.B. zur pH-Wert Änderung) und/oder physiologischen (z.B. antibakteriellen) Wirkungsweisen in die Rohrwandung eingebracht werden.

Eingebrachte radioaktive Partikel können z.B. der Vermeidung einer zu starken Proliferation bei der Gefäßheilung dienen. Chemische Partikel können z.B. bei Stents aus Polylactiden zu einer Pufferung des pH-Wertes beitragen, da das resorbierbare Material zu sauren Milchsäureabbauprodukten zerfällt.

Ebenso können zusätzlich kurze Fäden eines anderen polymeren oder metallischen Werkstoffes zur Festigkeitssteigerung in das Polymerrohr eingebracht werden. Diese festigkeitssteigernden Fäden können ebenfalls bioresorbierbar oder aber auch biostabil sein. Insbesondere wenn die zusätzlichen Fäden aus einem nicht biodegradierbaren Werkstoff bestehen, sollten die Fadenlängen vorzugsweise weniger als 50 µm, weiter bevorzugt weniger als 20 µm und besonders bevorzugt 10 Mikrometer und weniger betragen.

Grundsätzlich lassen sich durch die erfindungsgemäße Herstellung der Polymerrohre aus Fäden und der Zugabe von Additiven, Rohre mit vielfältigen Wirkmechanismen und Eigenschaften herstellen.

Die vorliegende Erfindung betrifft zudem **Verfahren zur Herstellung eines** porenfreien **Polymerrohrs,** bevorzugt eines (bio)resorbierbaren Polymerrohrs, umfassend mindestens die folgenden Schritte:
a) gerichtetes Aufwickeln mindestens eines Polymerfadens mit einem Durchmesser von ≤ 50 µm;
b) porenfreies Verschmelzen des mindestens einen aufgewickelten Polymerfadens bei oder bis maximal zu 15°C oberhalb der Schmelztemperatur des mindestens einen Polymerfadens.

Der Durchmesser des Polymerfadens ist dabei ≤ 50 µm und bevorzugt ≤ 30 µm, weiter bevorzugt ≤ 20 µm, weiter bevorzugt ≤ 15 µm, noch weiter bevorzugt ≤ 10 µm und besonders bevorzugt ≤ 2,5 µm. Die erfindungsgemäß verwendeten Polymerfäden sind bevorzugt Rundfasern, das heißt sie weisen einen runden Querschnitt auf, können jedoch auch andere Querschnitte, wie oval oder eckig aufweisen. Auch Polymerfäden mit einem hohlförmigen Querschnitt, d.h. Hohlfasern kommen in Frage.

Erfindungsgemäß ist es bevorzugt, wenn der mindestens eine Polymerfaden in Schritt a) auf einem sich drehenden Dorn aufgewickelt wird. Der Dorn weist bevorzugt einen konstanten Durchmesser auf, kann aber auch konisch oder wellenförmig ausgeformt sein, so dass der Durchmesser des Polymerrohrs bei gleicher Wandstärke über dessen Länge variiert. Alternativ kann über die Länge des Rohrs auch dessen Wandstärke variieren, so dass bei gleichbleibendem Innendurchmesser der Außendurchmesser variiert. Bei erfindungsgemäßen Polymerrohren, die für die Herstellung von Stents verwendet werden, ist es besonders bevorzugt, wenn zumindest der Innendurchmesser über die gesamte Länge des Polymerrohrs konstant ist.

Der mindestens eine Polymerfaden wird erfindungsgemäß gerichtet aufgewickelt. Dadurch erhalten in bestimmten Bereichen des Polymerrohrs oder in bestimmten Lagen oder über das gesamte Polymerrohr die Polymerfäden eine vorgegebene Ausrichtung, wobei dadurch wiederum die Molekülketten verschiedener Polymerfäden, welche entlang der Längsachse des jeweiligen Polymerfadens ausgerichtet sind, zueinander auch eine bestimmte Ausrichtung erhalten, wie z.B. konische Lagen um die Längsachse des Polymerrohrs. Durch dieses gerichtete Aufwickeln der Polymerfäden werden dem Polymerrohr vorteilhafte Eigenschaften verliehen wie beispielsweise eine höhere Zugfestigkeit sowie eine höhere Formstabilität im Nominaldurchmesser als auch eine Rückstellkraft eines deformierten Polymerrohres oder eines daraus hergestellten deformierten Stents, wobei diese Rückstellkraft das deformierte Polymerrohr als auch den deformierten Stent wieder auf den Nominaldurchmesser bringt, sei es aus einer überdehnten oder einer zusammengedrückten Form. Daher ist auch wichtig, dass das Polymerrohr bei der Herstellung bereits den Nominaldurchmesser besitzt, d.h. die Polymerfäden auf einem Dorn oder einer Spule aufgewickelt werden, welcher oder welche einen Außendurchmesser hat, der dem Innendurchmesser des Polymerrohrs oder des daraus hergestellten Stents entspricht, den das Polymerrohr oder der Stent nach der Dilatation im Gefäß haben soll.

Der Faden wird bevorzugt mit einer Ablegegeschwindigkeit von mindestens 100 m/min, weiter bevorzugt von mindestens 300 m/min und noch weiter bevorzugt von mindestens 600 m/min auf einem sich rotierenden Dorn abgelegt. Diese hohen Ablegegeschwindigkeiten sind einerseits durch die verfahrensbedingten hohen Geschwindigkeiten der Fadenherstellung vorteilhaft und andererseits erwünscht, um bei den sehr dünnen Fäden die Wicklungszeiten für ein Rohr gering zu halten. Für die bevorzugten Ablegegeschwindigkeiten des Fadens ergeben sich entsprechende, bevorzugte Rotationsgeschwindigkeiten des Dorns von mindestens 10.000 U/min, weiter bevorzugt mindestens 30.000 U/min, noch weiter bevorzugt mindestens 50.000 U/min und besonders bevorzugt mehr als 65.000 U/min.

Es ergibt sich für die Herstellung eines porenfreien Polymerrohrs und vorzugsweise eines bioresorbierbaren porenfreien Polymerrohrs ein bevorzugtes Verfahren, welches die folgenden Schritte umfasst:
a) gerichtetes Aufwickeln mindestens eines Polymerfadens mit einem Durchmesser von ≤ 50 µm auf einen sich drehenden Dorn zu einem Polymerrohr; und
b) porenfreies Verschmelzen des mindestens einen aufgewickelten Polymerfadens bei oder bis maximal zu 15^{o}C oberhalb der Schmelztemperatur des mindestens einen Polymerfadens.

Es ist zudem bevorzugt, wenn der Polymerfaden, welcher erfindungsgemäß gerichtet zu Polymerrohren aufgewickelt wird, durch Lösungsspinnen oder Schmelzspinnen hergestellt wird. Beim Lösungsspinnen wird das Polymer durch ein Lösungsmittel verflüssigt, beim Schmelzspinnen durch Erwärmung. Besonders bevorzugt sind erfindungsgemäße Verfahren, wobei der mindestens eine Polymerfaden durch Lösungsspinnen oder Schmelzspinnen hergestellt und gleichzeitig gerichtet auf einem sich drehenden Dorn aufgewickelt wird. Es ist auch bevorzugt, wenn es sich bei dem verwendeten Polymerfaden um einen unendlich fortlaufenden Faden oder Strang handelt. Dieser unendlich fortlaufende Polymerfaden wird nach Erreichen der gewünschten Wandstärke abgeschnitten.

Eine besonders bevorzugte Form des Lösungsspinnens ist das Elektrospinnen. Beim Elektrospinnen werden sehr dünne Fasern oder Fäden aus Polymerlösungen durch die Behandlung in einem elektrischen Feld hergestellt. Hierbei wird die Polymerlösung an einer Elektrode dosiert und durch das elektrische Feld von der Elektrode abgezogen und beschleunigt. Das Lösungsmittel verdampft bei dem extrem dünnen Faden sehr schnell und es kommt zu einer Ordnung der Polymerketten zu Kristalliten (Polymerkristallisation aus der Lösung).

Auf dem sich drehenden Dorn wird dabei der vorzugsweise unendlich fortlaufende Polymerfaden, bevorzugt aus resorbierbaren Polymeren, durch eine Apparatur gleichmäßig bis zu einer vorbestimmten Wandstärke gerichtet aufgewickelt. Man erhält ein aus einem Polymerfaden gewickeltes Rohr, wobei der Faden bzw. die Fäden idealerweise perfekt parallel zueinander liegen. Dabei ist es besonders bevorzugt, dass der mindestens eine Polymerfaden so aufgewickelt wird, dass eine Mantelfläche bzw. Wand des Polymerrohrs entsteht, die lückenlos bzw. durchgehend ist. Das erfindungsgemäße Polymerrohr weist daher bevorzugt keine Unterbrechung auf.

Die Poren zwischen den Fäden werden durch anschließendes **Verschmelzen** der Fäden geschlossen. Das Verschmelzen der Fäden kann so erfolgen, dass die Ausrichtung der Molekülketten nicht verloren geht. Es ist besonders bevorzugt, wenn das Verschmelzen des mindestens einen aufgewickelten Polymerfadens unter Vakuum stattfindet. Dies kann z.B. in einer Vakuumkammer erfolgen. Durch das Erzeugen des Vakuums wird die Luft aus den noch vorhandenen minimalen Zwischenräumen der eng beieinander liegenden Fäden eliminiert und die durch das Verschmelzen erzeugten Polymerrohre sind besonders porenfrei, so wie durch Extrusion oder Spritzgießen hergestellte Polymerrohre. Das Verschmelzen der gerichtet aufgewickelten Polymerfäden erfolgt im Schmelzbereich des eingesetzten Polymers oder in einem Bereich bis zu maximal 15°C, vorzugsweise bis zu maximal 10°C, weiter bevorzugt bis zu maximal 5°C oberhalb des Schmelzpunktes oder des Schmelzbereichs des eingesetzten Polymers und vorzugsweise auch nur für kurze Zeit wie beispielsweise 5 Sekunden bis 180 Sekunden, bevorzugt 20 Sekunden bis 120 Sekunden, so dass die Ausrichtung der Molekülketten erhalten bleibt. Die Molekülketten oder synonym als Polymerketten bezeichnet sind in dem Polymerfaden entlang der Längsachse des Polymerfadens ausgerichtet. Somit betrifft die vorliegende Erfindung ein weiteres bevorzugtes Verfahren zur Herstellung eines porenfreien Polymerrohrs und bevorzugt eines bioresorbierbaren porenfreien Polymerrohrs umfassend die folgenden Schritte:
a) gerichtetes Aufwickeln mindestens eines Polymerfadens mit einem Durchmesser von ≤ 50 µm vorzugsweise auf einen sich drehenden Dorn;
b) porenfreies Verschmelzen des mindestens einen aufgewickelten Polymerfadens bei oder bis maximal zu 15^{o}C oberhalb der Schmelztemperatur des mindestens einen Polymerfadens,
wobei der aufgewickelte mindestens eine Polymerfaden unter Beibehaltung der Ausrichtung der Molekülketten entlang der Fadenlängsachse porenfrei verschmolzen wird.

Ein weiterer Aspekt der vorliegenden Erfindung betrifft ein Verfahren zur Herstellung eines porenfreien Polymerrohrs und bevorzugt eines bioresorbierbaren porenfreien Polymerrohrs umfassend die folgenden Schritte:
a) gerichtetes Aufwickeln mindestens eines Polymerfadens mit einem Durchmesser von ≤ 50 µm vorzugsweise auf einen sich drehenden Dorn zu einem Polymerrohr;
b) porenfreies Verschmelzen des mindestens einen aufgewickelten Polymerfadens bei oder bis maximal zu 15^{o}C oberhalb der Schmelztemperatur des mindestens einen Polymerfadens im Vakuum.

Ein weiterer Aspekt der vorliegenden Erfindung betrifft ein Verfahren zur Herstellung eines porenfreien Polymerrohrs und bevorzugt eines bioresorbierbaren porenfreien Polymerrohrs umfassend die folgenden Schritte:
a) gerichtetes Aufwickeln mindestens eines Polymerfadens mit einem Durchmesser von ≤ 50 µm vorzugsweise auf einen sich drehenden Dorn;
b) porenfreies Verschmelzen des mindestens einen aufgewickelten Polymerfadens im Vakuum bei einer Temperatur innerhalb des Schmelzbereichs oder bis zu 15°C oberhalb des Schmelzbereichs des Polymers oder des aufgewickelten Polymerfadens.

Damit liegen die erfindungsgemäß hergestellten Polymerrohre bereits in kristalliner oder teilkristalliner Form vor, ohne dass ein Verstrecken erforderlich ist. Die Kristallinität wird sowohl durch Ausrichtung der Moleküle in den Polymerfäden als auch durch die gerichtete Aufwicklung des Polymerfadens / der Polymerfäden erzeugt. Eine bevorzugte Ausführungsform umfasst daher ein Polymerrohr hergestellt aus mindestens einem gerichtet aufgewickelten Polymerfaden wobei der Polymerfaden einen Durchmesser im Bereich von 50 nm bis 50 µm aufweist und die einzelnen Wicklungen des Polymerfadens, zumindest in vordefinierten Bereichen, parallel zueinander liegen. Diese Bereiche können z.B. einzelne Schichten von Wicklungen sein, aber auch Bereiche des Polymerrohrs aus denen später eine Strebe eines Stents entsteht und in denen die Richtung des Fadens bzw. der Winkel der Wicklung der späteren Beanspruchung der Strebe entspricht.

Der Begriff "Wicklung", wie hierein verwendet, bezeichnet dabei eine um eine Achse verlaufende Aufwicklung mindestens eines Polymerfadens. Mehrlagige Wicklungen bestehen aus konzentrischen Wendeln, die übereinander liegen. Alle Wicklungen des mindestens einen Polymerfadens, die in einer Ebene parallel zur Achse des Polymerrohrs verlaufen, bilden eine Schicht in der Wand des Polymerrohrs.

Das thermische Verschmelzen der gewickelten Polymerfäden kann erfindungsgemäß wie folgt durchgeführt werden:
Die in Schritt a) gerichtet aufgewickelten Polymerfäden können noch auf dem Dorn unter erhöhter Temperatur bei oder bis maximal zu 15°C oberhalb der Schmelztemperatur des mindestens einen Polymerfadens und additional unter Druck von außen zur Schließung der Poren verschmolzen werden. Der Druck kann z.B. durch eine außen anliegende Walze erzeugt werden.

Eine andere Methode ist das Aufdehnen des in Schritt a) gerichtet aufgewickelten Polymerrohres unter Temperatur bei oder bis maximal zu 15°C oberhalb der Schmelztemperatur des mindestens einen Polymerfadens und Druck von innen, wobei neben dem Verschmelzen die Fäden gleichzeitig gereckt werden. Dieses Verfahren ist dem Ballonblasen bei der Ballonkatheterherstellung ähnlich.

Es konnte überraschend gezeigt werden, dass die Zugfestigkeiten eines erfindungsgemäß hergestellten Polymerrohrs eine hohe Richtungsabhängigkeit zeigen (vgl. Fig. 2). Es wird vermutet, dass dies auf die unidirektional ausgerichteten Polymermoleküle zurückzuführen ist. Besonders vorteilhaft ist, dass zudem die Bruchdehnung in Richtung der Molekülketten trotz der hohen Zugfestigkeit mit über 100 % bei den erfindungsgemäßen Polymerrohren sehr hoch ist, d.h. die Verspannungen (innere Spannungen) des Materials sind gering. Dies ist ein grundsätzlicher, entscheidender Vorteil zu den durch Verstrecken verfestigten Rohren aus dem Stand der Technik, bei denen die Bruchdehnung für hohe Zugfestigkeiten vermindert ist.

Dennoch lässt sich auch bei einem erfindungsgemäß hergestellten Polymerrohr die erhöhte Festigkeit durch Verstrecken, d.h. Dehnen bzw. Verfestigen des Polymerrohrs weiter steigern. Bevorzugt sind dabei folgende Vorgehensweisen:
- Bereits beim Aufwickeln auf den Dorn können die Polymerfäden (mit den bereits ausgerichteten Molekülketten) gereckt werden.
- Eine Verfestigung kann durch Aufdehnen der gewickelten, aber noch nicht verschmolzenen Rohre erfolgen.
- Eine additionale Kombination aus beiden Verfahren (Recken des Fadens beim Aufwickeln und zusätzlich anschließendes Verstrecken des Rohres) ist möglich.
- Es ist möglich, das Verschmelzen der Fäden mit dem Verstrecken des gewickelten Rohres in einem thermomechanischen Vorgang zu kombinieren.
- Das fertig hergestellte Rohr (gewickelt und verschmolzen) lässt sich durch einen anschließenden Dehnvorgang verstrecken.

Ein bevorzugtes, erfindungsgemäßes Verfahren zur Herstellung eines Polymerrohrs umfasst somit, dass der mindestens eine Polymerfaden in Schritt a) beim gerichteten Aufwickeln auf den sich drehenden Dorn gereckt und damit verfestigt wird.

Ein weiteres bevorzugtes, erfindungsgemäßes Verfahren zur Herstellung eines Polymerrohrs ergibt sich dadurch, dass das Polymerrohr vor Schritt b), während des Verschmelzens gemäß Schritt b) oder nach Schritt b), auf einen Nenndurchmesser aufgedehnt und dabei verfestigt wird.

Daher ergeben sich die folgenden im Sinne der Erfindung bevorzugten Verfahren zur Herstellung eines Polymerrohrs:
Verfahren zur Herstellung eines porenfreien Polymerrohrs und bevorzugt eines bioresorbierbaren porenfreien Polymerrohrs, welches die folgenden Schritte umfasst:
a) gerichtetes Aufwickeln mindestens eines Polymerfadens mit einem Durchmesser von ≤ 50 µm auf einen sich drehenden Dorn zu einem Polymerrohr; und beim Aufwickeln Recken des mindestens einen Polymerfadens
b) porenfreies Verschmelzen des mindestens einen gerichtet aufgewickelten Polymerfadens bei oder bis maximal zu 15°C oberhalb der Schmelztemperatur des mindestens einen Polymerfadens.

Beim Recken setzt man beispielsweise die Polymerfäden zum Verformen unter Zugspannung, so dass sich die ungeordneten Polymere und die teilkristallinen Bereiche parallel zur Zugrichtung ausrichten.

Verfahren zur Herstellung eines porenfreien Polymerrohrs und bevorzugt eines bioresorbierbaren porenfreien Polymerrohrs, welches die folgenden Schritte umfasst:
a) gerichtetes Aufwickeln mindestens eines Polymerfadens mit einem Durchmesser von 50 µm zu einem Polymerrohr;
a') Verfestigung durch Aufdehnen des Polymerrohrs;
b) porenfreies Verschmelzen des mindestens einen gerichtet aufgewickelten Polymerfadens bei oder bis maximal zu 15°C oberhalb der Schmelztemperatur des mindestens einen Polymerfadens.

Verfahren zur Herstellung eines porenfreien Polymerrohrs und bevorzugt eines bioresorbierbaren porenfreien Polymerrohrs, welches die folgenden Schritte umfasst:
a) gerichtetes Aufwickeln mindestens eines Polymerfadens mit einem Durchmesser von ≤ 50 µm auf einen sich drehenden Dorn zu einem Polymerrohr; und beim Aufwickeln Recken des mindestens einen Polymerfadens;
a') Verfestigung durch Aufdehnen des Polymerrohrs;
b) porenfreies Verschmelzen des mindestens einen aufgewickelten Polymerfadens bei oder bis maximal zu 15°C oberhalb der Schmelztemperatur des mindestens einen Polymerfadens.

Verfahren zur Herstellung eines porenfreien Polymerrohrs und bevorzugt eines bioresorbierbaren porenfreien Polymerrohrs, welches die folgenden Schritte umfasst:
a) gerichtetes Aufwickeln mindestens eines Polymerfadens mit einem Durchmesser von ≤ 50 µm zu einem Polymerrohr;
b) porenfreies Verschmelzen des mindestens einen aufgewickelten Polymerfadens bei oder bis maximal zu 15°C oberhalb der Schmelztemperatur des mindestens einen Polymerfadens und parallel zum Verschmelzen Verstrecken des Polymerrohrs.

Verfahren zur Herstellung eines porenfreien Polymerrohrs und bevorzugt eines bioresorbierbaren porenfreien Polymerrohrs, welches die folgenden Schritte umfasst:
a) gerichtetes Aufwickeln mindestens eines Polymerfadens mit einem Durchmesser von ≤ 50 µm zu einem Polymerrohr;
b) porenfreies Verschmelzen des mindestens einen aufgewickelten Polymerfadens bei oder bis maximal zu 15°C oberhalb der Schmelztemperatur des mindestens einen Polymerfadens;
b') Verstrecken des verschmolzenen Polymerrohrs durch Dehnung.

Das Verstrecken des erfindungsgemäß hergestellten Polymerrohrs kann bei Raumtemperatur vorzugsweise aber bei erhöhter Temperatur erfolgen.

Zur Einstellung und/oder Fixierung der mechanischen Eigenschaften der erfindungsgemäßen Polymerrohre, insbesondere wenn ein Verstrecken stattgefunden hat, kann noch eine thermische Nachbehandlung durchgeführt werden. Durch Tempern bei Temperaturen unterhalb des Schmelzpunktes wird die Kristallinität erhöht. Durch Abschrecken können diese kristallinen Strukturen, aber auch die amorphen Strukturen fixiert werden.

Überraschend wurde gefunden, dass Polymerrohre, hergestellt durch gerichtete Aufwicklung möglichst dünner Polymerfäden, z.B. auf sich drehenden Dornen, eine erhöhte Festigkeit bei gleichzeitig optimaler Bruchdehnung aufweisen. In den dünnen Fäden mit Durchmessern im Mikrometerbereich oder Submikrometerbereich (mit einem

Durchmesser im Bereich von 50 nm bis 50 µm) sind die Moleküle bereits weitgehend unidirektional ausgerichtet.

Wie erläutert, führen die derzeitigen festigkeitssteigernden Methoden, angewendet bei Polymerrohren aus dem Stand der Technik, zu starken Einschränkungen bei der Verwendung als Ausgangsmaterial für Polymerstents. Die erfindungsgemäße Herstellung von Polymerr-Rohren beseitigt die starken Einschränkungen bzw. verbessert die Eigenschaften der Rohre, was insbesondere für deren Verwendung zur Herstellung von Stents und anderen Gefäßimplantaten entscheidend ist.

Beim Verstrecken (Dehnen) von Polymerrohren laufen grundsätzlich zwei Effekte parallel ab:
A) Die einzelnen langkettigen Moleküle werden in Dehnungsrichtung entknäuelt und gestreckt. In Richtung der Molekülketten ist die Festigkeit erhöht und quer zur Molekülrichtung geringer.
B) Beim Dehnen bewegen sich die Molekülketten aber auch zueinander, gleiten aufeinander ab und werden zueinander verspannt (Verfestigung). Es entstehen innere Spannungen. Mit zunehmenden inneren Spannungen nimmt die Dehnbarkeit des Werkstoffes ab.

Das Verstrecken ist daher nur begrenzt möglich, da Polymerrohre nur begrenzt ohne Bruchinduzierung gedehnt werden können. Zum Einen lassen sich die Moleküle nicht beliebig strecken und zum Anderen dürfen die inneren Spannungen die Bruchfestigkeit nicht überschreiten.

Erfindungsgemäß sollen die Vorgänge der Molekülausrichtung und der Verfestigung während der erfindungsgemäßen Herstellung der Polymerrohre völlig, zumindest aber weitgehend, getrennt voneinander ablaufen, um für beide Vorgänge ein Optimum zu erreichen.

Die Molekülausrichtung wird schon beim ersten Schritt der Herstellung der Polymerrohre, nämlich beim gerichteten Aufwickeln der Polymerfäden gewährleistet. Die Rohrherstellung erfolgt durch gerichtete Aufwicklung möglichst dünner Polymerfäden, bevorzugt auf schnelldrehenden Dornen oder Wellen oder Spulen. In den dünnen Fäden mit Durchmessern im Mikrometerbereich (≤ 50 µm) oder Submikrometerbereich (≤ 1 µm) sind die Moleküle bereits weitgehend unidirektional ausgerichtet. Die Fadenherstellung kann z.B. durch Extrusion aus kleinsten Spinndüsen (Schmelzspinnen) erfolgen oder vorzugsweise durch Lösungsspinnen und weiter bevorzugt durch Elektrospinnen. Eine Dehnung oder Verfestigung findet danach in einem oder mehreren weiteren Schritten statt.

Eine weitere bevorzugte Ausführungsform der vorliegenden Erfindung ist auf sogenannte **Hybrid-Polymerrohre** als auch daraus hergestellter Stents gerichtet. Ein Hybrid-Polymerrohr besteht aus mindestens zwei Polymeren mit unterschiedlichem Schmelzpunkt. Dabei liegt der Schmelzpunkt, der zwei enthaltenden Polymeren mindestens 10°C weiter bevorzugt mindestens 20°C auseinander. Die Formgebung der Rohrstruktur wird so vollzogen, wie zuvor beschrieben, allerdings erfolgt das Einbringen der Polymere wechselweise, wobei das Polymer mit dem höchsten Schmelzpunkt nicht als erstes und nicht als letztes aufgewickelt werden sollte. Eine Schicht besteht aus mindestens einer Fadenlage. Das Verschmelzen der Fäden wird bei Temperaturen oberhalb des Schmelzpunkts des Polymers mit dem niedrigeren Schmelzpunkt aber unterhalb des Schmelzpunkts des Polymers mit dem höheren Schmelzpunkt durchgeführt.

Durch die Erzeugung der unterschiedlichen Schichten aus Fäden und porenfrei verschmolzenen Polymerschichten werden die hohen Zugfestigkeiten von Fäden mit den Vorteilen von porenfreien Oberflächen verknüpft. Dadurch lassen sich Implantate, die aus diesen Hybrid-Polymerrohren bestehen wie konventionelle Implantate implantieren, verfügen aber gleichzeitig über höhere Festigkeiten. Weiterhin kann das Resorptionsverhalten über die Auswahl der Polymerschichten eingestellt werden. Die porenfreien Schichten halten das Rohr zusammen und sorgen für die Lastaufnahme quer zur Faserrichtung. Dahingegen erfolgt die Lastaufnahme in Faserrichtung durch die hochfesten und nicht verschmolzenen Polymerfasern.

Somit ist die vorliegende Erfindung auch auf Hybrid-Polymerrohre gerichtet, hergestellt durch gerichtete Aufwicklung oder schichtweise Aufwicklung mindestens eines Polymerfadens eines Polymers A, wobei der mindestens eine Polymerfaden des Polymers A einen Durchmesser von ≤ 50 µm aufweist und gerichtete Aufwicklung mindestens eines Polymerfadens eines Polymers B, wobei der mindestens eine Polymerfaden des Polymers B einen Durchmesser von ≤ 50 µm aufweist auf die Schicht aus dem mindestens einen Polymerfaden des Polymers A und gerichtete Aufwicklung mindestens eines Polymerfadens des Polymers A, wobei der mindestens eine Polymerfaden des Polymers A einen Durchmesser von ≤ 50 µm aufweist auf die Schicht aus dem mindestens einen Polymerfaden des Polymers B, wobei der mindestens eine Polymerfaden des Polymers A innerhalb einer Schicht aus Polymer A porenfrei miteinander verschmolzen wird bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

Anders formuliert betrifft die vorliegende Erfindung ein Hybrid-Polymerrohr, hergestellt durch gerichtete Aufwicklung oder schichtweise Aufwicklung von Polymerfäden eines Polymers A, wobei die Polymerfäden des Polymers A einen Durchmesser von ≤ 50 µm aufweisen und gerichtete Aufwicklung von Polymerfäden eines Polymers B, wobei die Polymerfäden des Polymers B einen Durchmesser von ≤ 50 µm aufweisen auf die Schicht aus den Polymerfäden des Polymers A und gerichtete Aufwicklung von Polymerfäden des Polymers A, wobei die Polymerfäden des Polymers A einen Durchmesser von ≤ 50 µm aufweisen auf die Schicht aus den Polymerfäden des Polymers B, wobei die Polymerfäden des Polymers A innerhalb einer Schicht aus Polymerfäden des Polymers A porenfrei miteinander verschmolzen werden bei einer Temperatur bei oder oberhalb des Schmelzpunktes des Polymers A aber bei oder unterhalb des Schmelzpunktes des Polymers B, wobei Polymer B mindestens einen um 10°C höheren Schmelzpunkt als Polymer A besitzt.

Nochmals anders formuliert betrifft die vorliegende Erfindung ein Hybrid-Polymerrohr aus mehreren Schichten gerichtet aufgewickelter und porenfrei miteinander innerhalb der jeweiligen Schicht verschmolzener Polymerfäden eines Polymers A und zwischen den Schichten aus gerichtet aufgewickelter und porenfrei miteinander verschmolzener Polymerfäden des Polymers A befindliche Schichten aus gerichtet aufgewickelten nicht miteinander innerhalb der jeweiligen Schicht verschmolzenen Polymerfäden eines Polymers B, wobei Polymer B mindestens einen um 10°C höheren Schmelzpunkt als Polymer A besitzt. Die porenfreie Verschmelzung der gerichtet aufgewickelten Polymerfäden des Polymers A innerhalb der jeweiligen Schicht erfolgt bei einer Temperatur oberhalb des Schmelzpunktes des Polymers A aber unterhalb des Schmelzpunktes des Polymers B.

Erfindungsgemäß erfolgt die Aufwicklung der Fäden des Polymers A als auch des Polymers B gerichtet. Wie bereits beschrieben, erfolgt die Aufwicklung der Polymerfäden auf einen sich drehenden Dorn oder einer sich drehenden Spule.

Dementsprechend betrifft die vorliegende Erfindung auch ein Verfahren zur Herstellung eines Hybrid-Polymerrohrs umfassend die folgenden Schritte:
a) gerichtetes Aufwickeln mindestens eines Polymerfadens eines Polymers A mit einem Durchmesser von ≤ 50 µm; und
b) gerichtetes Aufwickeln mindestens eines Polymerfadens eines Polymers B mit einem Durchmesser von ≤ 50 µm auf die Schicht aus dem mindestens einen Polymerfaden des Polymers A; und
c) gerichtetes Aufwickeln mindestens eines Polymerfadens des Polymers A mit einem Durchmesser von ≤ 50 µm auf die Schicht aus dem mindestens einen Polymerfaden des Polymers B; und
d) optional mehrfaches Wiederholen der Schritte b) und c); und
e) porenfreies Verschmelzen der mindestens einen aufgewickelten Polymerfäden aus Polymer A innerhalb der jeweiligen Schicht aus dem mindestens einen Polymerfaden aus Polymer A bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

Unter dieser Bedingungen werden die mindestens einen Polymerfäden innerhalb der jeweiligen Schichten aus mindestens einem Polymerfaden aus Polymer B nicht miteinander verschmolzen.

Alternativ betrifft die vorliegende Erfindung ein Verfahren zur Herstellung eines Hybrid-Polymerrohrs umfassend die folgenden Schritte:
a) gerichtetes Aufwickeln von Polymerfäden eines Polymers A mit einem Durchmesser von ≤ 50 µm; und
b) gerichtetes Aufwickeln von Polymerfädens eines Polymers B mit einem Durchmesser von ≤ 50 µm auf die Schicht aus den Polymerfäden des Polymers A; und
c) gerichtetes Aufwickeln von Polymerfäadens des Polymers A mit einem Durchmesser von ≤ 50 µm auf die Schicht aus den Polymerfäden des Polymers B; und
d) optional mehrfaches Wiederholen der Schritte b) und c); und
e) porenfreies Verschmelzen der aufgewickelten Polymerfäden innerhalb der jeweiligen Schicht aus den aufgewickelten Polymerfäden aus Polymer A bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

Ferner betrifft die vorliegende Erfindung ein Verfahren zur Herstellung eines Hybrid-Polymerrohrs umfassend die folgenden Schritte:
a) alternierendes gerichtetes Aufwickeln von Schichten von Polymerfäden eines Polymers A mit einem Durchmesser von ≤ 50 µm und von Schichten von Polymerfäden eines Polymers B mit einem Durchmesser von ≤ 50 µm, wobei das Polymer mit dem niedrigeren Schmelzpunkt die unterste als auch die oberste Schicht bildet, und
b) porenfreies Verschmelzen der Schichten aus den Polymerfäden aus Polymer A bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

Aus diesen Hybrid-Polymerrohren vorzugsweise mittels Laser geschnittene Stents haben besondere Eigenschaften, weil sie in den Schichten aus Polymer B weiterhin die vorteilhaften Eigenschaften der Polymerfäden ausnutzen und zusätzlich durch die porenfrei verschmolzenen Schichten aus Polymer A sehr gute Stabilität und Festigkeit besitzen. Die Hybrid-Polymerrohre sowie die daraus hergestellten Stents können auch aus mehr als 2 Polymeren hergestellt werden und können die hierin offenbarten Wirkstoffe und/oder Röntgenmarker enthalten. Somit können die Verfahren zur Herstellung eines Hybrid-Polymerrohrs noch den Schritt umfassen: Schneiden eines Stents aus dem Hybrid-Polymerrohr, vorzugsweise mittels Laser. Der Durchmesser des Hybrid-Polymerrohrs entspricht vorzugsweise dem Nenndurchmesser oder dem Nominaldurchmesser des Stents. Dementsprechend können die Verfahren zur Herstellung eines Hybrid-Polymerrohrs alternativ noch den Schritt umfassen: Schneiden eines Stents aus dem Hybrid-Polymerrohr, vorzugsweise mittels Laser, wobei der Durchmesser des Polymerrohrs dem Nenndurchmesser oder dem Nominaldurchmesser des Stents entspricht.

Ein weiterer Aspekt (nicht erfindungsgemäß) betrifft lasergeschnittene Stents aus Polymerrohren bestehend aus gerichtet aufgewickelten Polymerfäden, welche nicht miteinander porenfrei verschmolzen sind. Durch den Laserschnitt werden die geschnittenen Polymerfäden an der Schnittkante miteinander verschmolzen, so dass keine einzelnen Fäden sich aus dem fertig geschnittenen Stent ablösen können, aber aufgrund der erhaltenen Fadenstruktur der Stent vorteilhafte Eigenschaften hinsichtlich Zugfestigkeit und Rückstellkraft in Richtung des Nominaldurchmessers aufweist.

Die starke Richtungsabhängigkeit der Festigkeit der erfindungsgemäß hergestellten Rohre kann man insbesondere für die Herstellung von Gefäßimplantaten, vorzugsweise **Stents** und insbesondere bevorzugt bioresorbierbaren Stents nutzen. Unter Stents werden hierbei gitterförmige oder netzförmige Endoprothesen verstanden, die in ein Hohlorgan oder einen Körperhohlraum eingesetzt werden, um diesen offen zu halten. Das Grundgerüst eines Stents, womit hier die polymeren Streben ohne Beschichtung bezeichnet werden, bildet kein massives Rohr, sondern ein Gittergeflecht. Betrachtet man beispielsweise das Grundgerüst eines vaskulären Stents, so wird dieses aus einem massiven Rohr z.B. mittels Laser geschnitten, so dass sich einzelne, möglichst dünne Streben ergeben, welche untereinander verbunden sind. Die Anordnung und Ausformung der Streben und Knotenpunkte wird als Stentdesign bezeichnet. Im Sinne der vorliegenden Erfindung können alle üblichen Stentgeometrien verwendet werden.

Stents oder Gefäßstützen benötigen eine hohe radiale Festigkeit, um das Gefäß nach der Dilatation (Aufdehnung) offen zu halten. Hingegen ist in axialer Richtung, d.h. in Längsrichtung der Stents keine hohe Festigkeit gefordert. In radialer Richtung (Umfangsrichtung) ist zudem eine hohe Bruchdehnung gefordert, um den Stent bei Implantation mittels Ballonkatheter ausreichend und ohne Bruchgefahr dehnen zu können. Die erfindungsgemäß hergestellten Polymerrohre weisen genau diese Eigenschaften auf. Sie zeichnen sich durch eine hohe Festigkeit bei gleichzeitiger, hoher Bruchdehnung in radialer Richtung, d.h. in Umfangsrichtung, aus.

Ein bevorzugter Aspekt der vorliegenden Erfindung ist es daher Polymerrohre zur Verfügung zu stellen, die zur Herstellung eines Stents besonders gut geeignet sind. Diese Polymerrohre, vorzugsweise resorbierbaren Polymerrohre, können nach einem der hierin beschriebenen, erfindungsgemäßen Verfahren hergestellt werden.

Des Weiteren umfasst die vorliegende Erfindung daher einen Stent oder ein Gefäßimplantat, bevorzugt einen resorbierbaren Stent, hergestellt aus einem der hierin offenbarten Polymerrohre. Es handelt sich bei dem resorbierbaren Stent bevorzugt um einen Stent für Blutgefäße, Harnwege, Atemwege, Gallenwege oder den Verdauungstrakt. Unter diesen Stents sind wiederum die Stents für Blutgefäße oder allgemeiner für das Herz-Kreislaufsystem bevorzugt.

Beim Schneiden eines Stents werden Flächen zwischen den einzelnen Streben herausgeschnitten. Ein Stent oder ein vaskuläres Implantat weist daher eine Vielzahl von massiven Gerüstkomponenten (z.B. Streben in Form von Ringen, Spiralen, Wellen und Drähten) auf, sowie eine Vielzahl von Zwischenräumen zwischen diesen massiven Komponenten. Bei der gängigen Ausführungsform von Endoprothesen oder Stents laufen die Streben in Knotenpunkten zusammen. Es gibt jedoch auch Ausführungsformen von Endoprothesen, bei denen keine oder fast keine Knotenpunkte vorhanden sind und die Streben z.B. die Form von Ringen oder Spiralen haben. Bevorzugt handelt es sich um ballonexpandierbare Stents, welche mittels Katheter bis zur erkrankten oder zu behandelnden Stelle geschoben werden, wo die Stents auf ihren definierten Normdurchmesser aufgeweitet werden.

Ferner ist ein Kriechen des Materials, d.h. der gecrimpten bzw. im Gefäß dilatierten Stents unerwünscht, was durch eine Begrenzung der Dehnung bzw. der Verstreckung erzielt werden kann. Um unerwünschte Kriechvorgänge der implantierten Stents weitgehend zu vermeiden, hat es sich erfindungsgemäß als vorteilhaft erwiesen, Stents z.B. durch Laserschneiden im gedehnten Zustand herzustellen. Das bedeutet, die Stents werden aus erfindungsgemäßen Polymerrohren hergestellt, deren Durchmesser dem Nenndurchmesser, also dem Innendurchmesser des Stents im aufgeweiteten Zustand, entspricht. Danach kann der erfindungsgemäße Stent dennoch ohne Schwierigkeiten auf einen Katheterballon aufgebracht (gecrimpt) werden. Die Erfindung betrifft damit Stents hergestellt aus den erfindungsgemäßen Polymerrohren, wobei der Innendurchmesser des Polymerrohrs (des nicht aufgedehnten Polymerrohrs) dem Innendurchmesser des Stents nach Implantation entspricht.

Die erfindungsgemäßen Polymerrohre zur Herstellung eines Stents weisen daher vorzugsweise Innendurchmesser auf, die den aufgedehnten Stents entsprechen. Es ist daher bevorzugt, wenn das erfindungsgemäße Polymerrohr einen Innendurchmesser von 0,5 - 15 mm, weiter bevorzugt von 1 ― 10 mm und noch weiter bevorzugt von 2 ― 6 mm aufweist. Aus Polymerrohren dieser Durchmesser, die den Durchmessern dilatierter Stents entsprechen (Nenndurchmesser oder Nominaldurchmesser), werden die Gefäßimplantate bevorzugt lasergeschnitten. Damit liegen die hergestellten Stents im aufgedehnten (dilatierten) Zustand vor. Außerdem ist dies vorteilhaft, da im aufgedehnten Zustand die Stentstege oder Stentstreben vorzugsweise in Umfangsrichtung weisen und damit in Richtung der aufgewickelten Polymerfäden respektive in Richtung der Molekülketten bzw. der hohen Festigkeiten. Anschließend werden die Stents auf den Ballonkatheter gecrimpt, um sie implantieren zu können.

Die vorliegende Erfindung betrifft somit Verfahren zur Herstellung eines porenfreien Stents, bevorzugt eines resorbierbaren porenfreien Stents, umfassend die folgenden Schritte:
a) gerichtetes Aufwickeln mindestens eines Polymerfadens mit einem Durchmesser von ≤ 50 µm zu einem Polymerrohr;
b) porenfreies Verschmelzen des mindestens einen aufgewickelten Polymerfadens bei oder bis maximal zu 15^{o}C oberhalb der Schmelztemperatur des mindestens einen Polymerfadens; und
c) Schneiden eines Stents aus dem Polymerrohr, vorzugsweise mittels Laser.

Ein bevorzugtes Verfahren der vorliegenden Erfindung ist ein Verfahren zur Herstellung eines porenfreien Stents, bevorzugt eines resorbierbaren porenfreien Stents, umfassend die folgenden Schritte:
a) gerichtetes Aufwickeln mindestens eines Polymerfadens mit einem Durchmesser von ≤ 50 µm zu einem Polymerrohr;
b) porenfreies Verschmelzen des mindestens einen aufgewickelten Polymerfadens bei oder bis maximal zu 15^{o}C oberhalb der Schmelztemperatur des mindestens einen Polymerfadens; und
c) Schneiden eines Stents aus dem Polymerrohr, vorzugsweise mittels Laser, wobei der Durchmesser des Polymerrohrs dem Nenndurchmesser des Stents entspricht.

Die vorliegende Erfindung ist zudem auf ein Verfahren zur Herstellung eines porenfreien Stents und vorzugsweise eines bioresorbierbaren porenfreien Stents gerichtet, umfassend die folgenden Schritte:
a) gerichtetes Aufwickeln mindestens eines Polymerfadens mit einem Durchmesser von ≤ 50 µm vorzugsweise auf einen sich drehenden Dorn;
b) porenfreies Verschmelzen des mindestens einen aufgewickelten Polymerfadens bei oder bis maximal zu 15^{o}C oberhalb der Schmelztemperatur des mindestens einen Polymerfadens, wobei der gerichtet aufgewickelte mindestens eine Polymerfaden unter Beibehaltung der Ausrichtung der Molekülketten entlang der Fadenlängsachse porenfrei verschmolzen wird; und
c) Schneiden eines Stents aus dem Polymerrohr, vorzugsweise mittels Laser.

Ein weiterer Aspekt der vorliegenden Erfindung betrifft ein Verfahren zur Herstellung eines porenfreien Stents und vorzugsweise eines bioresorbierbaren porenfreien Stents umfassend die folgenden Schritte:
a) gerichtetes Aufwickeln mindestens eines Polymerfadens mit einem Durchmesser von ≤ 50 µm vorzugsweise auf einen sich drehenden Dorn zu einem Polymerrohr;
b) porenfreies Verschmelzen des mindestens einen gerichtet aufgewickelten Polymerfadens bei oder bis maximal zu 15^{o}C oberhalb der Schmelztemperatur des mindestens einen Polymerfadens im Vakuum; und
c) Schneiden eines Stents aus dem Polymerrohr, vorzugsweise mittels Laser.

Ein weiterer Aspekt der vorliegenden Erfindung betrifft ein Verfahren zur Herstellung eines porenfreien Stents und vorzugsweise eines bioresorbierbaren porenfreien Stents umfassend die folgenden Schritte:
a) gerichtetes Aufwickeln mindestens eines Polymerfadens mit einem Durchmesser von ≤ 50 µm vorzugsweise auf einen sich drehenden Dorn;
b) porenfreies Verschmelzen des mindestens einen gerichtet aufgewickelten Polymerfadens im Vakuum bei einer Temperatur innerhalb des Schmelzbereichs oder bis zu 15°C oberhalb des Schmelzbereichs des Polymers oder des aufgewickelten Polymerfadens; und
c) Schneiden eines Stents aus dem Polymerrohr, vorzugsweise mittels Laser.

Dabei treffen alle hierin gemachten Aussagen zu bevorzugten Ausführungsformen eines erfindungsgemäßen Verfahrens zur Herstellung eines Polymerrohrs entsprechend auch auf die Verfahren zur Herstellung des Stents zu.

Dieses Verfahren der Stentherstellung im gedehnten Zustand weist den weiteren Vorteil auf, dass unerwünschte Kriechvorgänge der Polymere weitgehend vermieden werden. Verformte Polymere neigen zum Kriechen in ihre Ursprungsform. Ein Stent, der im ungedehnten (gecrimpten) Zustand hergestellt und anschließend gedehnt wird, neigt zum Kriechen in Richtung seines ursprünglichen, kleineren Durchmessers. Dies ist für eine Stentimplantation nachteilig, da der implantierte Stent seinen Durchmesser im Gefäß wieder verringert. Dieses Verhalten wird bei den derzeitig zugelassenen, bioresorbierbaren Stents beobachtet.

Wird der Stent hingegen aus einem Polymerrohr mit einem großen Rohrdurchmesser, der dem dilatierten Gefäßdurchmesser entspricht, geschnitten, so wird er diesen Durchmesser nach der Dilatation im Gefäß beibehalten. Es ist erfindungsgemäß bevorzugt, wenn der Stent aus einem erfindungsgemäßen Polymerrohr mittels Laser ausgeschnitten wird. Dabei ist es weiter bevorzugt, wenn das erfindungsgemäße Polymerrohr dabei einen Durchmesser aufweist, welcher dem Durchmesser des aufgedehnten (bzw. aufgeweiteten oder inflatierten) Stents entspricht. Dabei entspricht der Innendurchmesser des aufgedehnten Stents dem Innendurchmesser des erfindungsgemäßen Polymerrohrs, welches zu seiner Herstellung verwendet wird. Das gleiche gilt für die Außendurchmesser, es sei denn der Stent wird nach der Herstellung aus dem Polymerrohr noch mit einer Beschichtung, z.B. einer wirkstofffreisetzenden Beschichtung, versehen.

Ein weiterer Vorteil der Herstellung der Stents aus Rohren deren Durchmesser dem aufgedehnten Durchmesser, d.h. Nenndurchmesser, des Stents entspricht ist, dass die Bruchgefahr bei einem Überdehnen über den Nenndurchmesser unkritischer ist als bei Stents, die aus kleineren Rohrdurchmessern (zumeist dem Durchmesser der gecrimpten, deflatierten Stents entsprechend) hergestellt wurden. Da die Festigkeit der Polymerrohre aus dem Stand der Technik zu Lasten der Dehnbarkeit optimiert wurde, sind die aus kleineren Rohrdurchmessern hergestellten Stents nur sehr begrenzt dehnbar und eine Überdehnung führt schnell zum Bruch. Bei den erfindungsgemäß hergestellten Stents sind bei gleicher, begrenzter Dehnbarkeit größere Gefäßdurchmesser erreichbar.

Es ist zudem möglich, die erfindungsgemäß hergestellten Polymerrohre noch zielgerichteter an die mechanischen Anforderungen von Gefäßimplantaten anzupassen:
Beim gerichteten Aufwickeln des Polymerfadens wird ein sich schnell drehender Dorn so hin- und her bewegt, dass der Faden z.B. kreuzweise unter einem bestimmten Winkel aufgewickelt wird. Das kreuzweise oder lagenweise Aufwickeln einer oder mehrerer Polymerfasern kann insbesondere beim Elektrospinnen auch durch die Ablenkung der Fasern in einem elektrischen Feld erfolgen.

Das gerichtete Aufwickeln kann gezielt im Hinblick auf die späteren Belastungen (Richtungen der Spannungsbeanspruchungen) der einzelnen Stege oder Streben des Stents nach Implantation, z.B. in ein Blutgefäß, geschehen. Die Fäden sollen dabei vorzugsweise in Richtung der höchsten Festigkeitsbeanspruchung liegen. Ferner ist es möglich, den Faden lagenweise d.h. in Schichten mit verschiedenen Winkeln aufzuwickeln und so gewünschte zielgerichtete Festigkeiten zu erreichen. Es ist auch möglich, in axialer Richtung des Rohres unterschiedliche Rohrwandstärken zu realisieren, die den unterschiedlichen Festigkeitsansprüchen eines Stents z.B. in der Mitte und an den Enden Rechnung trägt. Hierzu werden die Wicklungsstärken, d.h. die Anzahl der Wicklungen des Fadens in Längsrichtung auf dem Dorn variiert.

Ein anderes bevorzugtes Verfahren zur Herstellung eines Polymerrohrs gemäß der vorliegenden Erfindung, umfasst einen Schritt a) bei dem der Polymerfaden zielgerichtet lagen- und kreuzweise unter einem definierten Winkel aufgewickelt wird.

Daher ergibt sich für die Herstellung eines porenfreien Polymerrohrs und bevorzugt eines bioresorbierbaren porenfreien Polymerrohrs ein bevorzugtes Verfahren, welches die folgenden Schritte umfasst:
a) gerichtetes Aufwickeln mindestens eines Polymerfadens mit einem Durchmesser von ≤ 50 µm auf einen sich drehenden Dorn zu einem Polymerrohr, wobei der Dorn dabei so hin und her bewegt wird, dass der Polymerfaden unter einem bestimmten Winkel kreuzweise oder lagenweise aufgewickelt wird;
b) porenfreies Verschmelzen des mindestens einen gerichtet aufgewickelten Polymerfadens bei oder bis maximal zu 15°C oberhalb der Schmelztemperatur des mindestens einen Polymerfadens.

Ein weiteres bevorzugtes Verfahren für die Herstellung eines porenfreien Polymerrohrs und bevorzugt eines bioresorbierbaren porenfreien Polymerrohrs umfasst die folgenden Schritte:
a) gerichtetes Aufwickeln mindestens eines Polymerfadens mit einem Durchmesser von ≤ 50 µm auf einen sich drehenden Dorn zu einem Polymerrohr, wobei der Dorn dabei so hin und her bewegt wird, dass der Polymerfaden gemäß einem vorgegebenen Muster aufgewickelt wird;
b) porenfreies Verschmelzen des mindestens einen gerichtet aufgewickelten Polymerfadens bei oder bis maximal zu 15°C oberhalb der Schmelztemperatur des mindestens einen Polymerfadens.

Dabei ist es bevorzugt, wenn das Muster entsprechend dem Stentdesign gewählt wird und z.B. spiralförmig ist.

Ein weiteres bevorzugtes Verfahren für die Herstellung eines porenfreien Polymerrohrs und bevorzugt eines bioresorbierbaren porenfreien Polymerrohrs umfasst die folgenden Schritte:
a) gerichtetes Aufwickeln mindestens eines Polymerfadens mit einem Durchmesser von ≤ 50 µm zu einem Polymerrohr, wobei der mindestens eine Polymerfaden in mindestens zwei Schichten mit verschiedenen Winkeln aufgewickelt wird;
b) porenfreies Verschmelzen des mindestens einen gerichtet aufgewickelten Polymerfadens bei oder bis maximal zu 15°C oberhalb der Schmelztemperatur des mindestens einen Polymerfadens.

Es ist dabei besonders bevorzugt, wenn die Winkel entsprechend der Festigkeitsbeanspruchung der aus den Rohren hergestellten Bauteile gewählt werden. Im Fall eines Stents sind dies vor allem Radialkräfte, die auf die einzelnen Stentstreben einwirken. Daher ist es von besonderem Vorteil, dass die Winkel je nach Stentdesign frei wählbar sind.

Es ist bevorzugt, wenn die Wicklungsstärken, d.h. die Anzahl der übereinanderliegenden Wicklungen bzw. Schichten des Fadens, bei den erfindungsgemäß hergestellten Polymerrohren zwischen 5 Wicklungen und 1000 Wicklungen, weiter bevorzugt zwischen 10 Wicklungen und 600 Wicklungen, weiter bevorzugt zwischen 25 Wicklungen und 500 Wicklungen und noch weiter bevorzugt zwischen 50 Wicklungen und 300 Wicklungen liegt.

Die Aufwicklung der dünnen Polymerfäden erfordert hohe Ablagegeschwindigkeiten,d.h. hohe Rotationsgeschwindigkeiten des aufwickelnden rotierenden Dorns. Wird der Faden unter einem Winkel z.B. kreuzweise aufgewickelt, so sind schnelle Hinund Herbewegungen d.h. hohe axiale Verfahrgeschwindigkeiten des Dorns erforderlich.

Bei einem weiteren bevorzugten Verfahren zur Herstellung eines Polymerrohrs gemäß der vorliegenden Erfindung, werden im Schritt a) beim Aufwickeln der Fäden **Wirkstoffe** eingebracht. Es handelt sich dabei bevorzugt um Wirkstoffe, die zur Verminderung einer Restenose verwendet werden können. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-α-Hydroxy-Paclitaxel, Baccatin, Docetaxel, Biolimus A9, Myolimus, Novolimus, Pimecrolimus, Tacroliums, Temsirolimus, Zotarolimus, Everolimus, Ridaforolimus oder weiteren sogenannten Taxanen, bzw "Limus"-Derivaten.

Es können auch Wirkstoffkombinationen eingebracht werden. Dabei können die Wirkstoffe in unterschiedlichen Konzentrationen vorliegen. Die Konzentration eines verwendeten Wirkstoffs kann ebenfalls zwischen verschiedenen Teilbereichen des erfindungsgemäßen Polymerrohrs und damit des daraus herstellbaren Stents variieren. So kann im Bereich der Enden des Polymerrohrs mehr Wirkstoff enthalten sein, als im mittleren Bereich. Alternativ kann auf der luminalen (blutseitigen) Rohrinnenseite ein anderer Wirkstoff eingebracht werden als auf der abluminalen (gefäßseitigen) Rohraußenseite. Denkbar wären ein Wirkstoff zur Förderung der Endothelialisierung auf der luminalen Rohrinnenseite und ein Wirkstoff zur Proliferationshemmung auf der abluminalen Rohraußenseite.

Beim lagenweisen Aufwickeln von lösungs- oder schmelzgesponnenen Polymerfäden können Wirkstoffe zwischen den Polymerfäden aufgebracht werden. Damit ist es möglich, die Wirkstoffe direkt in die Wand des Polymerrohrs zu integrieren. Der Polymerfaden kann z.B. vor dem Aufwickeln mit einer Wirkstofflösung imprägniert werden. Eine besondere Möglichkeit der Einbeziehung von Wirkstoffen ergibt sich beim Lösungsspinnen. Beim Lösungsspinnen wird das vorzugsweise resorbierbare Polymer in einem Lösungsmittel in der Regel bei Raumtemperatur aufgelöst. In diese Lösung kann der Wirkstoff mit eingebracht werden, sofern er mit dem Lösungsmittel verträglich ist. Damit befindet sich der Wirkstoff bereits in dem lösungsgesponnenen Faden, der zu einem Rohr aufgewickelt wird.

Beim anschließenden Verschmelzen (Verbacken) der Polymerrohre sind die Temperaturen deutlich niedriger und damit wirkstoffschonender als beim Extrudieren oder Spritzgießen. Beim Extrudieren bzw. Spritzgießen der Polymerrohre werden die Schmelztemperaturen der Polymere deutlich überschritten.

Auch kann ein Wirkstoffgradient über die Wandstärke des Polymerrohrs vorteilhaft sein. Hierzu können z.B. Polymerfäden mit unterschiedlicher Wirkstoffkonzentration bzw. mit unterschiedlichen Wirkstoffen zur Herstellung des Polymerrohrs verwendet werden. Alternativ können einem kontinuierlichen Polymerfaden bei dessen Herstellung unterschiedliche Wirkstoffkonzentrationen bzw. unterschiedliche Wirkstoffe zugesetzt werden. In Kombination mit dem mindestens einen Wirkstoff oder der Wirkstoffkombination können weitere Substanzen z.B. Transportvermittler eingebracht werden.

Als Lösungsmittel eignen sich bevorzugt organische Lösungsmittel wie beispielsweise Chloroform (Trichlormethan), Methylenchlorid (Dichlormethan), Tetrafluorethylen (TFE), Hexafluoroisopropanol (HFIP), Tetrahydrofuran (THF), Aceton (Dimethylketon), Diethylether, Methanol, Ethanol, Propanol, Isopropanol, Diethylketon, Dimethylformamid (DMF), Dimethylacetamid, Essigsäureethylester, Dimethylsulfoxid (DMSO), Benzol, Toluol, Xylol, t-Butylmethylether (MTBE), Cyclohexan, N-Methyl-2-Pyrrolidone, Pentan, Hexan, Heptan, wobei Methylenchlorid und Chloroform bevorzugt sind.

Die spätere Freisetzung der Wirkstoffe erfolgt mit der Implantation der Polymerrohre bzw. der daraus hergestellten Implantate wie Stents. In der Anfangsphase nach der Implantation von Stents, hergestellt aus den erfindungsgemäßen Polymerrohren, finden Zersetzungsvorgänge der Polymermoleküle statt, ohne dass der Stent degradiert, d.h. ohne Masseverluste. Die Wirkstoffabgabe erfolgt im Wesentlichen durch Diffusion. Bei der später einsetzenden Degradation erfolgt die Wirkstoffabgabe durch Erosion. Möglich ist auch, die Wirkstoffe in unterschiedlichen Konzentrationen in die Rohrwand einzubringen und damit dem Verlauf der Diffusion und der Degradation des Stents und/oder dem Heilungsprozess des Gefäßes Rechnung zu tragen. Im Gegensatz dazu wird bei beschichteten, resorbierbaren und nicht resorbierbaren Polymerstents erst die Wirkstoffbeschichtung abgebaut und danach der Stent, ohne das noch Wirkstoffe vorhanden sind.

Dennoch ist es auch möglich die erfindungsgemäß hergestellten Polymerrohre bzw. Polymerstents mit einer zusätzlichen Beschichtung zu versehen. Dabei handelt es sich bevorzugt um eine wirkstofffreisetzende Beschichtung. Der Wirkstoff oder eine Wirkstoffkombination kann dabei alleine oder in einer geeigneten Matrix auf die polymere Oberfläche der Rohre oder Stents aufgebracht werden. Eine geeignete Matrix kann ein Polymer, bevorzugt ein resorbierbares Polymer, sein. Das Polymer der wirkstofffreisetzenden Beschichtung kann dabei mit dem Polymer des Grundgerüsts identisch sein.

Mit dem Wirkstoff zusammen können auch weitere Substanzen aufgebracht werden, die dessen Freisetzung bzw. Haftung auf dem Stent beeinflussen. Als pharmakologisch verträgliche Träger in einer wirkstofffreisetzenden Beschichtung können Substanzen dienen, ausgewählt aus der Gruppe bestehend aus oder umfassend: Stärke, Natrium-Carboxymethylstärke, Sorbitol, Sucrose, Magnesiumstearat, Dicalciumphosphat, Calciumsulfat, Talk, Mannitol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Isopropylmyristate und - palmitate, Citrate, wie Tributyl- und Triethylcitrate und deren Acetylderivate, Phtalate wie Dimethylphtalat oder Dibutylphtalat, Benzoesäurebenzylester, Triacetin, 2-Pyrrolidon, Agar, Cellulose, Cellulosederivate wie Methylcellulose, Natrium-Carboxymethylcellulose und Hydroxypropylmethylcellulose,.

Die wirkstofffreisetzende Beschichtung kann mittels bekannter Verfahren wie beispielsweise Sprühverfahren, Tauchverfahren, Plasmaverfahren, Pinselverfahren, Spritzenverfahren, Elektrospinnen oder Pipettierverfahren auf das Grundgerüst (bestehend aus Stentstreben, die aus dem erfindungsgemäßen Polymerrohr geschnitten wurden) aufgebracht werden.

Aus den obigen Ausführungen wird deutlich, dass die Eigenschaften der erfindungsgemäßen Polymerrohre auch Eigenschaften der aus diesen Rohren hergestellten Stents bzw. Gefäßstützen sind. Somit sollen alle hierin angeführten Merkmale und Merkmalskombinationen der erfindungsgemäßen Polymerrohre auch im Zusammenhang mit den erfindungsgemäßen Stents verstanden werden. Sowohl die Polymerrohre als auch die Stents werden in ihrer Charakteristik vor allem durch die erfindungsgemäßen Herstellungsverfahren geprägt. Die vorliegende Erfindung betrifft demnach auch Polymerrohre und Stents erhältlich nach einem der erfindungsgemäßen Verfahren. Die Polymerrohre und Stents hergestellt durch eins der erfindungsgemäßen Verfahren zeichnen sich durch eine erhöhte radiale Festigkeit bei gleichzeitig hoher oder sogar erhöhter Bruchdehnung aus, wobei die radiale Festigkeit bevorzugt > 80 MPa, weiter bevorzugt > 90 MPa, noch weiter bevorzugt > 100 MPa beträgt und die Bruchdehnung > 30 %, weiter bevorzugt > 40 %, noch weiter bevorzugt > 50 % ist.

### Figurenbeschreibung

- **Figur** 1:: Figur 1 zeigt einen Vergleich zwischen Spannungs-Dehnungs-Diagrammen aus dem Stand der Technik und erfindungsgemäß hergestellten Rohren aus PLLA Kurve 1, 2: Spannungs-Dehnungs-Diagramm eines PLLA Rohres (3,3 x 0.15 mm) erfindungsgemäß hergestellt gemäß Beispiel 6 und mit beschriebenen Spiralen (s. Figur 4) getestet um die Radialspannungen zu bestimmen. Kurve 1 und 2 stellen jeweils einen Prüfkörper dar. Kurve 3, 4: Spannungs-Dehnungs-Diagramm eines orientierten PLLA Rohres (3.4 x 0.15 mm) aus dem Stand der Technik vom Marktführer für extrudierte Rohre für medizinische Anwendungen. Mit den beschriebenen Spiralen getestet um die Radialspannungen zu bestimmen. Kurve 3 und 4 stellen jeweils einen Prüfkörper dar. Kurve 5: Spannungs-Dehnungs-Diagramm eines orientierten PLLA Rohres (3.4 x 0.15 mm) aus dem Stand der Technik vom Marktführer für extrudierte Rohre für medizinische Anwendungen. Mit den beschriebenen Streifen getestet um die Axialspannungen zu bestimmen Kurve 6, 7: Spannungs-Dehnungs-Diagramm eines PLLA Rohres (3,3 x 0.15 mm) erfindungsgemäß hergestellt und mit beschriebenen Streifen getestet um die Axialspannungen zu bestimmen. Kurve 6 und 7 stellen jeweils einen Prüfkörper dar.
- **Figur 2:**: Figur 2 zeigt einen Vergleich zwischen Spannungs-Dehnungs-Diagrammen aus dem Stand der Technik und erfindungsgemäß hergestellten Rohren aus PCL. Kurve 1, 2: Spannungs-Dehnungs-Diagramm eines PCL Rohres (3,3 x 0.15 mm) erfindungsgemäß hergestellt und mit beschriebenen Spiralen getestet um die Radialspannungen zu bestimmen. Kurve 1 und 2 stellen jeweils einen Prüfkörper dar. Kurve 3, 4: Spannungs-Dehnungs-Diagramm eines PCL Rohres (3,0 x 0.15 mm) erfindungsgemäß hergestellt und mit beschriebenen Streifen getestet um die Axialspannungen zu bestimmen. Kurve 3 und 4 stellen jeweils einen Prüfkörper dar. Kurve 5: Spannungs-Dehnungs-Diagramm eines PCL Rohres (3.4 x 0.15 mm) aus dem Stand der Technik vom Marktführer für extrudierte Rohre für medizinische Anwendungen. Mit den beschriebenen Streifen getestet.
- **Figur 3:**: Zeigt den Versuchsaufbau für die Zugversuche gemäß Figur 1 und 2. Die Traverse wird über den Spindelhub nach oben verfahren. Durch die Dehnung des Prüfkörpers entsteht eine Kraft, die mithilfe der kalibrierten Kraftmessdose aufgezeichnet wird.
- **Figur 4:**: Zeigt wie die Polymerrohre mit dem Laser geschnitten werden, um die Rohrstücke für die Zugversuche gemäß Figur 1 und 2 zu erhalten. Diese Probenkörper werden für orientierte, also anisotrope Polymere verwendet, damit Spannungen in radialer als auch in axialer Richtung aufgenommen werden können.

### Beispiele

### Beispiel 1: Herstellung eines erfindungsgemäßen Polymerrohrs

Ein erfindungsgemäßes Polymerrohr wurde hergestellt, indem das hochreine Polymergrundmaterial Polycaprolacton (PCL) in Tetrafluorethylen (TFE) gelöst wird und zwar 170 mg PCL pro 1 ml TFE und im Elektrospin-Verfahren zu einem ca. 2 µm dünnen Faden gezogen wird. Der Faden wird mit hoher Geschwindigkeit von ca. 700 m/min von der Elektrospindüse abgezogen. Dieser Prozess wird mit einem Polymerfördervolumen von 10ml/h bei einer Spannung von 25kV durchgeführt. Das Tetrafluorethylen verdampft augenblicklich aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen.

Anschließend werden die eng nebeneinander liegenden Fäden des Polymerrohres bei einer Temperatur oberhalb der Schmelztemperatur bei ca. 65°C verschmolzen. Um ein porenfreies Verschmelzen und eine hohe Dichte der Rohrwandung sicherzustellen, wird beim Verschmelzen von außen eine mechanische Kraft auf das Rohr ausgeübt, d.h. das Rohr wird auf den Dorn gepresst.

### Beispiel 2: Herstellung eines erfindungsgemäßen Stents

Nach Abkühlung wird das gemäß Beispiel 1 hergestellte Rohr vom Dorn gezogen und in eine Laseranlage eingespannt. Mit einem Ultrakurzpulslaser wird das Stentdesign in das Polymerrohr geschnitten. Die Ausschnitte fallen heraus, so dass nur die Stentstruktur bestehend aus den einzelnen Stentstegen übrig bleibt. Nach Reinigungsschritten ist der Stent fertig, um auf einen Ballonkatheter gecrimpt und anschließend implantiert zu werden.

### Beispiel 3: Herstellung eine erfindungsgemäßen Polymerrohrs enthaltend Paclitaxel

Wenn der Stent Wirkstoffe freisetzen soll, um das Heilungsverhalten zu verbessern, so kann der Wirkstoff beispielsweise direkt bei der Herstellung ins Polymerrohr eingebracht werden.

Hierzu wurde in die Tetrafluorethylenlösung des Polymers aus Beispiel 1 Paclitaxel in zwei verschiedenen Konzentrationen mit hinzu gemischt (1:10 und 1:3 Paclitaxel zu Polymer). Da das Polymerrohr lagenweise über die Rohrwandstärke zwei verschiedene Wirkstoffkonzentrationen enthalten sollte, wird zunächst unter Verwendung der ersten Lösung im Elektrospin-Verfahren ein ca. 0,75 µm dünner Faden gezogen und dieser auf einem mit hoher Geschwindigkeit drehenden Dorn aufgewickelt. Dabei wird der Dorn hin und her bewegt, um eine gleichmäßige Aufwicklung zu erzielen. Nach 200 Lagen wird das Aufwickeln des Fadens unterbrochen und mit einem Faden aus der Lösung mit der zweiten Wirkstoffkonzentration fortgesetzt bis insgesamt 300 Lagen aufgewickelt sind. Das Verfahren der Fadenverschmelzung änderte sich gegenüber Beispiel 1 nicht. Auch das Verfahren des Laserschneidens des Stents wie in Beispiel 2 beschrieben ändert sich durch den Wirkstoff nicht.

Sofern sich die Wirkstoffkonzentration über die Rohrlänge ändern soll, kann ebenfalls mit Fäden unterschiedlicher Wirkstoffkonzentration gearbeitet werden.

### Beispiel 4: Herstellung eines erfindungsgemäßen Polymerrohrs enthaltend Sirolimus

Ein erfindungsgemäßes Polymerrohr wurde hergestellt, indem das hochreine Polymergrundmaterial Polyglycolide (PGA) in Hexafluoroisopropanol (HFIP) gelöst wird und zwar 80 mg PGA pro 1 ml HFIP und im Elektrospin-Verfahren zu einem ca. 1 µm dünnen Faden gezogen wurde. Der Faden wird mit hoher Geschwindigkeit von ca. 800 m/min von der Elektrospinndüse abgezogen. Das Hexafluoroisopropanol verdampft unmittelbar aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen. Dieser Prozess wird ca. 30 Minuten lang mit einem Polymerfördervolumen von 10ml/h durchgeführt. Zur Funktionalisierung wird das Polymerrohr während dieses Prozess mit Medikamenten beladen. Dazu wird gegen Ende des Prozesses nach 20 Minuten, nach 25 Minuten und nach 28 Minuten der Dorn mit dem Wirkstoff Sirolimus besprüht. Dabei wird die Konzentration jeweils um 20% erhöht, um in der obersten Schicht eine hohe Konzentration zu erhalten und dann im Verlauf der späteren Elutionszeit eine abnehmende Konzentration freizusetzen.

Anschließend werden die eng nebeneinander liegenden Fäden des Polymerrohres bei einer Temperatur oberhalb der Schmelztemperatur bei ca. 235°C verschmolzen. Um ein porenfreies Verschmelzen und eine hohe Dichte der Rohrwandung sicherzustellen, wird der Verschmelzungsprozess im Vakuum bei 10 bis 500 nBar durchgeführt.

### Beispiel 5: Herstellung eines erfindungsgemäßen röntgensichtbaren Polymerrohrs

Ein erfindungsgemäßes Polymerrohr wurde hergestellt, indem das hochreine Polymergrundmaterial Polyhydroxybutyrate (PHB) in Chloroform (CHCl₃) gelöst wird und zwar 40 mg PHB pro 1 ml CHCl₃. Zur Funktionalisierung wird die Polymerlösung mit röntgensichtbaren Partikeln beladen. Dazu werden 10mg Nanopartikel aus Tantal-Nitrid pro ml Lösung hinzugefügt. Im Elektrospin-Verfahren wird das Polymer zu einem ca. 500 nm dünnen Faden gezogen. Die Nanopartikel haften an den Fäden und werden in die Faserstruktur aufgenommen. Der Faden wird mit hoher Geschwindigkeit von ca. 750 m/min von der Elektrospinndüse abgezogen. Das Chloroform verdampft unmittelbar aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen. Dieser Prozess wird ca. 30 Minuten lang mit einem Polymerfördervolumen von 10ml/h durchgeführt.

Anschließend werden die eng nebeneinander liegenden Fäden des Polymerrohres bei einer Temperatur oberhalb der Schmelztemperatur bei ca. 185°C verschmolzen. Um ein porenfreies Verschmelzen und eine hohe Dichte der Rohrwandung sicherzustellen, wird der Verschmelzungsprozess im Vakuum bei 10 bis 500 nBar durchgeführt.

### Beispiel 6: Versuchsdurchführung zu Figur 1

### a) Herstellung des PLLA Rohres

Kurve 1, 2, 6, 7 erfindungsgemäßes Rohr, das wie folgt hergestellt wurde: Das hochreine Polymergrundmaterial Poly-L-Iactid (PLLA) wird in Chloroform (CHCl₃) gelöst wird und zwar 60 mg PLLA pro 1 ml Chloroform und im Elektrospin-Verfahren zu einem ca. 0,8 µm dünnen Faden gezogen wird. Der Faden wird mit hoher Geschwindigkeit von ca. 800 m/min von der Elektrospindüse abgezogen. Dieser Prozess wird mit einem Polymerfördervolumen von 8ml/h bei einer Spannung von 22kV durchgeführt. Das Chloroform verdampft augenblicklich aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen.

Anschließend werden die eng nebeneinander liegenden Fäden des Polymerrohres bei einer Temperatur oberhalb der Schmelztemperatur bei ca. 185 °C verschmolzen. Um ein porenfreies Verschmelzen und eine hohe Dichte der Rohrwandung sicherzustellen, wird das Verschmelzen im Hochvakuum bei 30nBar durchgeführt. Nach Abkühlung wird das hergestellte Rohr vom Dorn gezogen.

Kurve 3, 4, 5 sind von einem kommerziell erhältlichen PLLA Rohr hergestellt nach dem folgenden Verfahren:
Die PLLA Rohre wurden nach dem Stand der Technik hergestellt. Dazu wurde der HAAKE MiniLab II Micro Compounder der Firma Thermofisher Scientific mit zuvor getrocknetem PLLA Granulat PL38 der Firma Purac beladen. Die Extrusion wurde nach vom Hersteller angegeben Standards durchgeführt. Die extrudierten Rohre wurden sichtkontrolliert und mit dem Laser in Abschnitte von jeweils 300 mm geschnitten. Diese Rohrabschnitte wurden in die Ballonformmaschine 503 der BW-Tec AG mit einer Form mit einem Innendurchmesser von 3,4 mm und einer Länge von 140 mm eingespannt. Danach wurden die Rohre mit 12 bar beaufschlagt und die Form auf eine Temperatur 30°C oberhalb der Glasübergangstemperatur erwärmt. Im Anschluss wurden die Rohre aus der Form entfernt und erneut sichtkontrolliert.

Beide Rohre, d.h. das erfindungsgemäße und das aus dem Stand der Technik wurden gleichermaßen in die Spannzange einer Laseranlage eingespannt. Mit einem Ultrakurzpulslaser wird das Prüfkörperdesign (Figur 4) in das zu testende Polymerrohr geschnitten. Die Ausschnitte fallen heraus, so dass nur die Prüfkörper übrig bleiben. Der Streifen (s. Figur 4 rechter Teil), dient zu Prüfung der mechanischen Eigenschaften in axialer Richtung. Die Spirale wird zu Prüfung in radialer Richtung (s. Figur 4 linker Teil), verwendet. Die Spirale verläuft nicht vollständig in radialer Richtung, sondern hat eine leichte Steigung. Diese Steigung kann aber bei der Bestimmung der Radialkraft vernachlässigt werden, da die sich ergebende Abweichung unter 1% liegt und die Steigung dazu führt, dass die maximale Spannung eher unterschätzt als überschätzt wird. Die Streifen haben eine einheitliche Breite von 2 mm und eine Länge von 40 mm. Die Dicke der Streifen entspricht der Wandstärke des Ausgangsrohrs. Die Prüfkörper werden unter dem Lichtmikroskop kontrolliert, nummeriert abgelegt und unmittelbar nach dem Laserschneiden wird der Zugversuch durchgeführt.

### b) Durchführung der Zugversuche für Kurven

Die Zugversuche werden bei Raumtemperatur zwischen 20°C und 25°C durchgeführt. Die erzeugten Prüfkörper werden in eine Zugprüfmaschine Zwick Z005 mittels flacher mechanischer Spannpratzen eingespannt. Die Streifen liegen bereits ein einer flachen Form vor, sodass sie zunächst oben zwischen die Pratzen geschoben werden und die obere Pratze über zwei Spannschrauben fixiert gespannt wird. Danach wird die Traverse nach unten verfahren, bis die Probe in die untere Pratze geschoben werden kann. Wiederum wird die Pratze mittels zweier Spannschrauben geschlossen. Die Spiralen werden genauso gezogen wie die Streifen, allerdings müssen diese zuvor begradigt werden. Dazu wird ein Ende der Spirale zunächst in den oberen Halter eingespannt und dann mit der Pinzette nach unten aufgezogen und entknäuelt in den unteren Halter geschoben. Im Zugversuch wird dann eine Vorspannung von 5 MPa aufgebracht, um die Probe zu begradigen. Im Anschluss wird die jeweilige Probe mit einer konstanten Geschwindigkeit von 5 mm / min auseinandergezogen. Eine kalibrierte Kraftmessdose mit einer Maximalkraft von 5 kN zeichnet die dadurch entstehende Kraft auf. In der zugehörigen Software wird die Kraft durch den Ausgangsquerschnitt geteilt und so die Spannung, die in Figur 1 dargestellt ist, errechnet. Die Dehnung auf der x-Achse wird über den Verfahrweg der Traverse ermittelt. Der Versuchsaufbau ist in Figur 3 gezeigt.

### Beispiel 7: Versuchsdurchführung zu Figur 2

Ein Rohr wurde gemäß Beispiel 1 hergestellt. Ein weiteres herkömmlich extrudiertes Rohr (Figure 2, Kurve 5) wurde so wie die kommerziell erhältlichen Rohre hergestellt. Dazu wurde der HAAKE MiniLab II Micro Compounder der Firma Thermofisher Scientific mit zuvor getrocknetem PCL Granulat PC12 der Firma Purac beladen. Die Extrusion wurde nach vom Hersteller angegeben Standards durchgeführt. Die extrudierten Rohre wurden sichtkontrolliert und mit dem Laser in Abschnitte von jeweils 300mm geschnitten. Diese Rohrabschnitte wurden in die Ballonformmaschine 503 der BW-Tec AG mit einer Form mit einem Innendurchmesser von 3,4 mm und einer Länge von 140 mm eingespannt. Danach wurden die Rohre mit 12 bar beaufschlagt. Im Anschluss wurden die Rohre aus der Form entfernt und erneut sichtkontrolliert.

Die Rohre, d.h. das erfindungsgemäße und das aus dem Stand der Technik, werden wie in Beispiel 6 geprüft. Da das herkömmliche Rohr isotrop ist, ergeben beide Prüfkörper aus dem herkömmlichen Rohr gleiche Ergebnisse, sodass hier nur eine Kurve dargestellt ist.

### Beispiel 8: Herstellung eines erfindungsgemäßen Hybrid-Polymerrohrs

Ein erfindungsgemäßes Hybrid-Polymerrohr wurde wie folgt hergestellt: Das hochreine Polycaprolacton (PCL) wird in Tetrafluorethylen (TFE) gelöst und zwar 170 mg PCL pro 1 ml TFE. Ein weiteres hochreines, hochmolekulargewichtiges Polymer (PLLA) wird in Chloroform gelöst und zwar 60mg PLLA pro 1ml Chloroform. Im Elektrospin-Verfahren wird das PCL zu einem ca. 1 µm dünnen Faden gezogen. Der Faden wird mit hoher Geschwindigkeit von ca. 800 m/min von der Elektrospinndüse abgezogen. Das TFE verdampft unmittelbar aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen. Nach 10 übereinanderliegenden Faserlagen wird der Dorn in eine zweite Elekrospinnanlage eingespannt. Im zweiten Elektrospin-Verfahren wird das PLLA zu einem ca. 700 nm dünnen Faden gezogen. Der Faden wird ebenfalls mit hoher Geschwindigkeit von ca. 800 m/min von der Elektrospinndüse abgezogen. Das Chloroform verdampft unmittelbar aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen. Erneut werden 10 übereinanderliegende Faserlagen erzeugt. Die Abfolge der beiden Elektrospinnverfahren wird 15 mal wiederholt. Wobei die erste und die letzte Schicht immer aus dem Polymer mit dem niedrigeren Schmelzpunkt, hier also PCL besteht. Anschließend werden die eng nebeneinander liegenden Fäden des Polymerrohres bei einer Temperatur oberhalb der Schmelztemperatur von PCL bei ca. 70°C verschmolzen. Dadurch verschmelzen die PCL Fäden porenfrei miteinander. Um ein porenfreies Verschmelzen und eine hohe Dichte der Rohrwandung sicherzustellen, wird der Verschmelzungsprozess im Vakuum bei 10 bis 500 nBar durchgeführt. Die PLLA Fäden werden bei 70°C nicht verschmolzen und bleiben als Fäden erhalten. Dadurch wird die hohe Festigkeit, die von Fasern bekannt ist, ausgenutzt. Durch das Vakuum werden die PLLA Fäden möglichst stark aneinandergedrückt, sodass das freie nicht-lasttragende Volumen reduziert werden kann. Die Fäden werden durch die Schichten aus PCL in Lage gehalten und sorgen dafür, dass beim späteren Laserschneiden, dass Rohr nicht auseinanderfällt. Aus diesem Hybrid-Rohr lassen sich Stents schneiden, die mit gewöhnlichen Ballonkathetern implantierbar sind. Die PLLA Fasern sorgen für eine hohe Radialfestigkeit während die porenfreien Schichten aus PCL die Lasten in axialer Richtung aufnehmen. Da PCL bei Körpertemperatur im Glasbereich vorliegt, besteht hier großes Potenzial zur Verformung, was sich wiederum positiv auf axiale Biegefähigkeit (auch Conformability genannt) auswirkt. Das führt dazu, dass der Stent die Arterie offen hält, sie aber nicht unnötig begradigt.

## Patentansprüche

1. Porenfreies Polymerrohr hergestellt durch gerichtete Aufwicklung mindestens eines Polymerfadens, wobei der mindestens eine Polymerfaden einen Durchmesser von ≤ 50 µm aufweist und der gerichtet aufgewickelte mindestens eine Polymerfaden bei oder bis maximal zu 15°C oberhalb der Schmelztemperatur des mindestens einen Polymerfadens porenfrei verschmolzen ist.

2. Porenfreies Polymerrohr gemäß Anspruch 1, wobei das porenfreie Polymerrohr bioresorbierbar ist.

3. Porenfreies Polymerrohr gemäß Anspruch 1 oder 2, wobei das Polymerrohr hergestellt ist aus mindestens einem Polymer ausgewählt aus der Gruppe bestehend aus:
Poly(ε-caprolacton), Polyurethan, Polyhydroxybutyrat, Polylactonsäure, Polyglycolsäure, Polylactid, Polyglycolid, Copolymere der Polylactide und Polyglycolide.

4. Porenfreies Polymerrohr gemäß einem der Ansprüche 1 - 3, wobei das Polymerrohr über seine Länge eine unterschiedliche Anzahl an Wicklungen bzw. unterschiedliche Wanddicken aufweist.

5. Porenfreies Polymerrohr gemäß einem der Ansprüche 1 - 4, wobei das Polymerrohr mindestens einen Wirkstoff und/oder mindestens einen Röntgenmarker umfasst.

6. Hybrid-Polymerrohr hergestellt durch gerichtete Aufwicklung oder schichtweise gerichtete Aufwicklung mindestens eines Polymerfadens eines Polymers A, wobei der mindestens eine Polymerfaden des Polymers A einen Durchmesser von ≤ 50 µm aufweist und gerichtete Aufwicklung mindestens eines Polymerfadens eines Polymers B, wobei der mindestens eine Polymerfaden des Polymers B einen Durchmesser von ≤ 50 µm aufweist auf die Schicht aus dem mindestens einen Polymerfaden des Polymers A und gerichtete Aufwicklung mindestens eines Polymerfadens des Polymers A, wobei der mindestens eine Polymerfaden des Polymers A einen Durchmesser von ≤ 50 µm aufweist auf die Schicht aus dem mindestens einen Polymerfaden des Polymers B, wobei der mindestens eine Polymerfaden des Polymers A innerhalb einer Schicht aus Polymer A porenfrei miteinander verschmolzen wird bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

7. Verfahren zur Herstellung eines porenfreien Polymerrohrs umfassend die folgenden Schritte:
a) gerichtetes Aufwickeln mindestens eines Polymerfadens mit einem Durchmesser von ≤ 50 µm; und
b) porenfreies Verschmelzen des mindestens einen gerichtet aufgewickelten Polymerfadens bei oder bis maximal zu 15°C oberhalb der Schmelztemperatur des mindestens einen Polymerfadens.

8. Verfahren zur Herstellung eines porenfreien Polymerrohrs gemäß Anspruch 7, wobei der mindestens eine Polymerfaden durch Lösungsspinnen, Elektrospinnen oder Schmelzspinnen hergestellt wird.

9. Verfahren zur Herstellung eines porenfreien Polymerrohrs gemäß Anspruch 7 oder 8, wobei der mindestens eine Polymerfaden in Schritt a) auf einem sich drehenden Dorn aufgewickelt wird.

10. Verfahren zur Herstellung eines porenfreien Polymerrohrs gemäß einem der Ansprüche 7 ― 9, wobei das Polymerrohr den Nominaldurchmesser ausweist.

11. Verfahren zur Herstellung eines porenfreien Polymerrohrs gemäß einem der Ansprüche 7 - 10, wobei der aufgewickelte mindestens eine Polymerfaden unter Beibehaltung der Ausrichtung der Molekülketten entlang der Fadenlängsachse bei oder bis maximal zu 15°C oberhalb der Schmelztemperatur des mindestens einen Polymerfadens porenfrei verschmolzen ist.

12. Verfahren zur Herstellung eines Hybrid-Polymerrohrs umfassend die folgenden Schritte:
a) gerichtetes Aufwickeln mindestens eines Polymerfadens eines Polymers A mit einem Durchmesser von ≤ 50 µm; und
b) gerichtetes Aufwickeln mindestens eines Polymerfadens eines Polymers B mit einem Durchmesser von ≤ 50 µm auf die Schicht aus dem mindestens einen Polymerfaden des Polymers A; und
c) gerichtetes Aufwickeln mindestens eines Polymerfadens des Polymers A mit einem Durchmesser von ≤ 50 µm auf die Schicht aus dem mindestens einen Polymerfaden des Polymers B; und
d) optional mehrfaches Wiederholen der Schritte b) und c); und
e) porenfreies Verschmelzen der mindestens einen gerichtet aufgewickelten Polymerfäden aus Polymer A innerhalb der jeweiligen Schicht aus dem mindestens einen Polymerfaden aus Polymer A bei einer Temperatur bei oder oberhalb des Schmelzpunktes von Polymer A aber bei oder unterhalb des Schmelzpunktes von Polymer B, wobei Polymer B mindestens einen um 10°C höheren Schmelzpunkt als Polymer A besitzt.

13. Stent hergestellt aus einem Polymerrohr gemäß einem der Ansprüche 1 ― 6.

## Claims

1. Pore-free polymer tube prepared by directional winding of at least one polymer filament, wherein the at least one polymer filament has a diameter of ≤ 50 µm and the directionally wound at least one polymer filament is melted pore-free at or maximal at 15°C above the melting temperature of the polymer filament.

2. Pore-free polymer tube according to claim 1, wherein the pore-free polymer tube is bioresorbable.

3. Pore-free polymer tube according to claims 1 or 2, wherein the polymer tube is prepared from at least one polymer selected from a group consisting of:
poly(ε-caprolactone), polyurethane, polyhydroxybutyrate, polylactonic acid, polyglycolic acid, polylactide, polyglycolide, copolymers of the polylactides and polyglycolides.

4. Pore-free polymer tube according to one of the claims 1 - 3, wherein the polymer tube exhibits a different number of windings or differential wall thickness along its length.

5. Pore-free polymer tube according to one of the claims 1 - 4, wherein the polymer tube comprises at least one active agent and/or at least one radiographic marker.

6. Hybrid polymer tube prepared by directional winding or layer-wise directional winding of at least one polymer filament of a polymer A, wherein the at least one polymer filament of the polymer A has a diameter of ≤ 50 µm, and directional winding of at least one polymer filament of a polymer B on top of the layer of the at least one polymer filament of polymer A, wherein the at least one polymer filament of the polymer B has a diameter of ≤ 50 µm, and directional winding of at least one polymer filament of polymer A on top of the layer of the at least one polymer filament of polymer B, wherein the at least one polymer filament of the polymer A has a diameter of ≤ 50 µm, , wherein the at least one polymer filament of polymer A within the layer of polymer A is melted together pore-free at a temperature at or above the melting point of polymer A but at or below the melting point of polymer B, wherein polymer B has an at least 10°C higher melting point compared to polymer A.

7. Method for the preparation of a pore-free polymer tube comprising the following steps:
a) directional winding of at least one polymer filament with a diameter of ≤ 50 µm; and
b) pore-free melting of the at least one directionally wound polymer filament at or maximal at 15°C above the melting temperature of the at least one polymer filament.

8. Method for the preparation of a pore-free polymer tube according to claim 7, wherein the at least one polymer filament is prepared by solution spinning, electrospinning, or melting spinning.

9. Method for the preparation of a pore-free polymer tube according to claims 7 or 8, wherein in step a) the at least one polymer filament is wound on a rotating mandrel.

10. Method for the preparation of a pore-free polymer tube according to one of the claims 7 ― 9, wherein the polymer tube has the nominal diameter.

11. Method for the preparation of a pore-free polymer tube according to one of the claims 7 - 10, wherein the wound at least one polymer filament is melted pore-free at or maximal at 15°C above the melting temperature of the at least one polymer filament while maintaining the alignment of the molecular chains along the longitudinal axis of the filament.

12. Method for the preparation of a hybrid polymer tube comprising the following steps:
a) directional winding of at least one polymer filament of a polymer A with a diameter of ≤ 50 µm; and
b) directional winding of at least one polymer filament of a polymer B with a diameter of ≤ 50 µmon top of the layer of the at least one polymer filament of polymer A; and
c) directional winding of at least one polymer filament of polymer A with a diameter of ≤ 50 µmon top of the layer of the at least one polymer filament of polymer B; and
d) optional multiple repetition of the steps b) and c); and
e) pore-free melting of the at least one directionally wound polymer filament of polymer A within the respective layer of the at least one polymer filament of polymer A at a temperature at or above the melting point of polymer A but at or below the melting point of polymer B, wherein polymer B has an at least 10°C higher melting point compared to polymer A.

13. Stent prepared of a polymer tube according to one of the claims 1 - 6.

## Revendications

1. Un tube en polymère non poreux préparé par l'enroulement directionnel d'au moins un filament de polymère, où ledit filament de polymère a un diamètre inférieur ou égal à 50 µm et le filament de polymère enroulé de façon directionnelle est fusionné de façon non poreuse à la température de fusion du filament de polymère ou à un maximum de 15 °C au-dessus de cette température de fusion.

2. Un tube en polymère non poreux conformément à la revendication 1, celui-ci étant biorésorbable.

3. Un tube en polymère non poreux conformément à l'une des revendications 1 ou 2, préparé à partir d'au moins un polymère sélectionné parmi : poly(ε-caprolactone), polyuréthane, polyhydroxybutyrate, acide polylactonique, acide polyglycolique, polylactide, polyglycolide, copolymères de polylactides et polyglycolides.

4. Un tube en polymère non poreux conformément à l'une des revendications 1 à 3, présentant un nombre variable d'enroulements ou une épaisseur de paroi variable sur sa longueur.

5. Un tube en polymère non poreux conformément à l'une des revendications 1 à 4, comprenant au moins un agent actif et/ou au moins un marqueur radiographique.

6. Un tube en polymère hybride obtenu par enroulement directionnel ou enroulement directionnel par couche d'au moins un filament de polymère A ayant un diamètre inférieur ou égal à 50 µm, et l'enroulement directionnel d'au moins un filament de polymère B par-dessus la couche constituée d'au moins un filament de polymère A, où les filaments de polymère B ont un diamètre inférieur ou égal à 50 µm, et l'enroulement directionnel d'au moins un filament de polymère A par-dessus la couche constituée d'au moins un filament de polymère B, où les filaments de polymère A ont un diamètre inférieur ou égal à 50 µm, où au moins un filament de polymère A à l'intérieur de la couche de polymère A est fusionné de façon non poreuse à une température égale ou supérieure à la température de fusion du polymère A, mais inférieure à la température de fusion du polymère B, ladite température de fusion du polymère B étant supérieure d'au moins 10 °C à celle du polymère A.

7. La méthode de préparation d'un tube en polymère non poreux comprenant les étapes suivantes :
a) enroulement directionnel d'au moins un filament de polymère d'un diamètre inférieur ou égal à 50 µm ; et
b) fusion non poreuse d'au moins un filament de polymère enroulé de façon directionnelle, à la température de fusion dudit filament de polymère ou à un maximum de 15 °C au-dessus de cette température de fusion.

8. La méthode de préparation d'un tube en polymère non poreux conformément à la revendication 7, où le filament de polymère est préparé par filature, électrofilature ou filature par fusion.

9. La méthode de préparation d'un tube en polymère non poreux conformément à l'une des revendications 7 ou 8, où au moins un filament de polymère est enroulé sur un mandrin rotatif à l'étape a).

10. La méthode de préparation d'un tube en polymère non poreux conformément à l'une des revendications 7 à 9, où le tube en polymère est de diamètre nominal.

11. La méthode de préparation d'un tube en polymère non poreux conformément à l'une des revendications 7 à 10, où le filament de polymère enroulé est fusionné de façon non poreuse à la température de fusion dudit filament de polymère ou à un maximum de 15 °C au-dessus de cette température de fusion, en maintenant l'alignement des chaînes moléculaires le long de l'axe longitudinal du filament.

12. La méthode de préparation d'un tube de polymère hybride comprenant les étapes suivantes :
a) enroulement directionnel d'au moins un filament de polymère A d'un diamètre inférieur ou égal à 50 µm ; et
b) enroulement directionnel d'au moins un filament de polymère B d'un diamètre inférieur ou égal à 50 µm par-dessus la couche constituée d'au moins un filament de polymère A ; et
c) enroulement directionnel d'au moins un filament de polymère A d'un diamètre inférieur ou égal à 50 µm par-dessus la couche constituée d'au moins un filament de polymère B ; et
d) répétition (éventuellement plusieurs répétitions) des étapes b) et c) ; et
e) fusion non poreuse d'au moins un filament de polymère A enroulé de façon directionnelle avec la couche correspondante constituée d'au moins un filament de polymère A, à la température de fusion du polymère A ou à une température supérieure à cette température de fusion, mais inférieure à la température de fusion du polymère B, celle-ci étant supérieure d'au moins 10 °C à la température de fusion du polymère A.

13. Un stent préparé à partir d'un tube en polymère conformément à l'une des revendications 1 à 6.
